# EUROPEAN PATENT APPLICATION

(11) **EP 3 069 609 A1**
(43) Date of publication of application: **21.09.2016**
(21) Application number: 16161026.6
(22) Date of filing: 18.03.2016
(51) Int. Cl.: A01N 1/00, A01N 1/02, A61K 35/52

(54) **INCORPORATION OF PHOSPHATIDYLCHOLINE IN A MEDIA COMPOSITION**

(30) Priority: 20.03.2015 US 201562135753 P
(71) Applicant: Dow AgroSciences LLC, Indianapolis IN 46268-1054 (US)
(72) Inventor: GARRISON, Robbi L., Filmore, IN 46128 (US)
(74) Representative: f & e patent

(57) **Abstract**

The present disclosure provides the use of a phosphatidylcholine compound as a component of a media composition. The resulting media composition can be used for the cryopreservation of eukaryotic cells. The cryopreserved eukaryotic cells can be thawed and recovered for inoculation and/or growth in a media to reproduce new cells. Provided herein are compositions and methods for the cryopreservation of eukaryotic cells in a media composition containing a phosphatidylcholine compound.

## Description

### CROSS REFERENCE TO RELATED APPLICATION

The present application claims priority to the benefit of U.S. Provisional Patent Application Ser. No. 62/135,753 filed Mar. 20, 2015, the disclosure of which is hereby incorporated by reference in its entirety.

### BACKGROUND OF THE INVENTION

Cryopreservation compositions and methods are used for the preservation and storage of eukaryotic cells under low-temperature conditions. Such cryopreservation techniques require that the eukaryotic cells are protected from any biological damage that may occur during exposure to the low-temperature conditions. Further, the exposure of the eukaryotic cells to such conditions should not impair or alter the eukaryotic cells upon thawing and recovery. Nor, should the eukaryotic cells exhibit any altered physiological or genetic modifications upon the application of the cryopreservation technique and the exposure to the low-temperature conditions. Despite the establishment of traditional cryopreservation techniques over the years, the need for novel cryopreservation techniques -- especially novel cryopreservation techniques for recalcitrant eukaryotic cells -- are necessary and desirable.

Unfortunately, traditional methods of cryopreservation and the use of known media compositions for cryopreservation are not successful for all eukaryotic cell lines. The skilled artisan recognizes the challenges and difficulties associated with preserving any eukaryotic cell line, and especially the recalcitrant eukaryotic cell line. Most biological materials, including eukaryotic cell lines, cannot survive freezing and thawing from cryogenic temperatures without cryoprotective agents and procedures. A number of known cryopreservatives possess properties which can protect a cell from the damaging effects of cryogenic freezing. The essence of such cryopreservatives is to effect cell dehydration and concentration of the cytosol in a controlled and minimally injurious manner so that ice crystallization in the cytosol is precluded or minimized during cooling and/or freezing. However, even these known cryopreservatives may not be effective for the cryopreservation of recalcitrant cell lines. Culturing of eukaryotic cells, for example plant cells, pose special problems for current cryopreservation technology. Prolonged culturing of the cells can result in a loss of biosynthetic ability which had been present in the original isolates. Furthermore, the cells may present phenotypic alterations that directly result from the culturing of the cells under low-temperature conditions.

The establishment of *in vitro* collections of eukaryotic cells and tissue under normal or limited growth conditions can be maintained in the laboratory. However, *in vitro* tissue culturing requires the continuous need of subculturing established cell lines in cell suspension media under regimented growth conditions. Over time, such cultures may become contaminated by microbial organisms and may undergo physiological or genetic changes over the long-term. For long-term storage, elimination of routine subculturing is desirable because of concerns with mutation, contamination, labor cost, and risk of human error associated with tissue culture.

Therefore, a need exists for development of improved cryopreservation techniques, and to develop compositions of matter that allow for successful cryopreservation and recovery of eukaryotic cells without any physiological or genetic modifications to the eukaryotic cells. Such improved cryopreservation techniques allow for the reduction of microbial contamination, minimization of genetic change, avoidance of aging in finite cell lines, and minimization of physiological change in the eukaryotic cell lines.

### BRIEF SUMMARY OF THE INVENTION

In an embodiment, the disclosure relates to a media composition, comprising an aqueous solution including at least one cryopreservation agent, a cell suspension media, and a phosphatidylcholine; and, a eukaryotic cell. In other embodiments, the cryopreservation agent is selected from the group consisting of glycerol, DMSO (dimethyl sulfoxide), propylene glycol, ethylene glycol, acetamide, and methanol. In further embodiments, the cryopreservation agent is provided at concentrations from 0.5% to 75% of the total volume of the media composition. For example, the cell suspension media comprises a dicotyledonous plant cell suspension media or a monocotyledonous plant cell suspension media. In other embodiments, the eukaryotic cell comprises a plant cell. Exemplary plant cells include monocotyledonous plant cells and a dicotyledonous plant cells. Further, the monocotyledonous plant cell can be a maize, wheat, rice, black grass, tall fescue, bent grass, and anza wheat plant cell. Likewise, the dicotyledonous plant cell is selected from the group consisting of soybean, tobacco, Arabidopsis, canola, cotton, rape, sunflower, and carrot plant cell. In other embodiments, the plant cell comprises a plant suspension cell line, or a photo-autotrophic suspension cell line. Also included are embodiments to plant cells that comprise a plant tissue or a plant structure. Exemplary plant tissues and plant structures include embryos, meristematic tissues, leaves, pollen, roots, root tips, anther, silks, flowers, kernels, bulbs, tubers, rhizomes, calli, and seeds thereof. In further examples the plant cell comprises a transgene.

Furthermore, the subject disclosure relates to phosphatidylcholine selected from the group consisting of soybean L-α-phosphatidylcholine, rape seed L-α-phosphatidylcholine, cotton seed L-α-phosphatidylcholine, and sunflower seed L-α-phosphatidylcholine. In some embodiments, the phosphatidylcholine is provided at concentrations from 0.05% to 50% of the total volume of the media composition.

In addition, the subject disclosure relates to cooling of the media composition to reach a state of freezing. Accordingly, the media composition comprises a temperature of about 4°C to about -196°C.

In an embodiment, the disclosure relates to a method for cryopreserving a plurality of eukaryotic cells, the method comprising the steps of: suspending the plurality of eukaryotic cells in a media composition; acclimating the plurality of eukaryotic cells and media composition to a reduced temperature; and, freezing the plurality of eukaryotic cells and media composition to a temperature of less than 4°C. In other embodiments, the media composition comprises an aqueous solution including at least one cryopreservation agent, a cell suspension media, and a phosphatidylcholine. In yet other embodiments, the plurality of eukaryotic cells comprise plant cells. In still further embodiments, the plant cells are selected from the group consisting of monocotyledonous plant cells and dicotyledonous plant cells. In some instances the plant cells are transgenic plant cells. In some embodiments, the plurality of eukaryotic cells in a media composition allows diffusion of the media composition molecules into the eukaryotic cell membrane or cell wall. Also considered as an embodiment of acclimating is lowering the plurality of eukaryotic cells and media composition to a reduced temperature. As such, the plurality of eukaryotic cells and media composition are lowering to a temperature for a pre-determined amount of time to a pre-determined temperature to result in the frozen plurality of eukaryotic cells and media composition. In some embodiments, the plurality of eukaryotic cells and media composition are acclimated to a reduced temperature comprises lowering the temperature of the aqueous solution by flash-freezing to result in the frozen plurality of eukaryotic cells and media composition. In other embodiments, the temperature of the frozen plurality of eukaryotic cells and media composition are raised for a pre-determined amount of time to a pre-determined temperature, thereby returning the plurality of eukaryotic cells to the pre-cryopreservation state with minimal damage to the structural integrity of said eukaryotic cell.

Further considered in the disclosure are the steps of growing the plurality of eukaryotic cells on culture media; inoculating a culture flask containing culture media with the plurality of eukaryotic cells to form a liquid culture and passaging the liquid culture of eukaryotic cells at least 1, 2, 3, 4, 5, 6, 7, 8, 9, or 10 times; and recovering said passaged plurality of eukaryotic cells. In some embodiments, the method further comprises the step of adding the recovered plurality of eukaryotic cells to an aqueous solution to form a media composition. In other embodiments, the frozen plurality of eukaryotic cells and media composition are thawed. In still further embodiments, the plurality of eukaryotic cells from the media composition are recovered and cultured.

In an embodiment, the disclosure relates to a cryopreservation solution comprising at least one cryopreservation agent and a phosphatidylcholine. In some embodiments, the cryopreservation solution further comprises a eukaryotic cell, wherein the eukaryotic cell is suspended in a cell suspension media. Accordingly, the cryopreservation agent can be selected from a penetrating cryopreservation agent and a non-penetrating cryopreservation agent. In some embodiments, the penetrating cryopreservation agent comprises glycerol and/or DMSO. In embodiments, the cryopreservation agent is provided at concentrations from 0.05% to 50% of the total volume of the cryopreservation solution. In other embodiments, the cryopreservation solution diffuses within the cell membrane or cell wall of the eukaryotic cell. In certain embodiments, the eukaryotic cell comprises a plant cell. The plant cells can be selected from the group consisting of a monocotyledonous plant cell and a dicotyledonous plant cell. Exemplary monocotyledonous plant cells include maize, wheat, rice, black grass, tall fescue, bent grass, and anza wheat. Exemplary dicotyledonous plant cells include soybean, tobacco, Arabidopsis, canola, cotton, rape, sunflower, and carrot. The monocotyledonous and dicotyledonous plant cell may comprise a plant suspension cell line, or a photo-autotrophic suspension cell line. In some embodiments, the plant cell comprises a plant tissue or a plant structure. Exemplary plant tissues or the plant structures include embryos, meristematic tissues, leaves, pollen, roots, root tips, anther, silks, flowers, kernels, bulbs, tubers, rhizomes, calli, and seeds thereof. In some instances the plant cell may comprise a transgene.

Furthermore, the subject disclosure relates to phosphatidylcholine selected from the group consisting of soybean L-α-phosphatidylcholine, rape seed L-α-phosphatidylcholine, cotton seed L-α-phosphatidylcholine, and sunflower seed L-α-phosphatidylcholine. In some embodiments, the phosphatidylcholine is provided at concentrations from 0.05% to 50% of the total volume of the media composition.

In addition, the subject disclosure relates to cooling of the media composition to reach a state of freezing. Accordingly, the media composition comprises a temperature of about 4°C to about -196°C.

In an embodiment, the disclosure relates to a method for suspending phosphatidylcholine within at least one cryopreservation agent to produce a cryopreservation solution.

In addition to the exemplary aspects and embodiments described above, further aspects and embodiments will become apparent by study of the following descriptions.

### DETAILED DESCRIPTION

### I. Overview

Disclosed herein is a novel media composition containing an aqueous solution and a eukaryotic cell. The aqueous solution further comprises a cell suspension media mixed with a cryopreservation solution, wherein the cryopreservation solution contains phosphatidylcholine suspended with at least one cryopreservation agent. Various types of cryopreservation agents can include penetrating and non-penetrating cryopreservation agents. For example, the following cryopreservation agents may be used as described by the disclosure; glycerol, DMSO (dimethyl sulfoxide), propylene glycol, ethylene glycol, acetamide, and methanol. Differing types of eukaryotic cells may be cryopreserved. For example, the compositions and methods of the disclosure are applicable to mammalian cells, plant cells (monocotyledonous, dicotyledonous, and algae), yeast cells, and other cell types containing an enveloped nucleus and other cellular organelles within a cell plasma membrane. Furthermore, the cells may be differentiated or undifferentiated. Plant suspension cell lines, photo-autotrophic suspension cell lines, plant tissues, and plant structures are exemplary, non-limiting embodiments of the disclosure. In some instances, the cells contain transgenes stably integrated within the genome of the chloroplast, mitochondria, or nucleus.

The media composition is cooled and/or frozen such that the media composition reaches a temperature of about 4°C to about -196°C. Typically, an aqueous solution is suspended with a plurality of eukaryotic cells and allowed to acclimate before the cooling/freezing results in a temperature of less than 4°C. The media composition (comprising a plurality of eukaryotic cells mixed with the aqueous solution) is allowed to acclimate such that the molecules of the media composition diffuse into the eukaryotic cell membrane or cell wall. Next, the media composition is cooled/frozen for a pre-determined amount of time to a pre-determined temperature (e.g., gradual cooling or flash-freezing) to result in the frozen plurality of eukaryotic cells and aqueous solution. After the freezing has been completed the media composition (comprising the plurality of eukaryotic cells mixed with the aqueous solution) can be stored at a temperature of less than 4°C for an indeterminate amount of time.

Subsequently, the media composition (comprising the plurality of eukaryotic cells mixed with the aqueous solution) can be recovered for use by thawing. Accordingly, the thawing entails raising the temperature of the frozen plurality of eukaryotic cells and aqueous solution for a pre-determined amount of time to a pre-determined temperature, thereby returning the plurality of eukaryotic cells to the pre-cryopreservation state with minimal damage to the structural integrity of the eukaryotic cell. The thawed eukaryotic cells may be cultured. As such, the plurality of eukaryotic cells are grown on culture media and then inoculated into a culture flask containing culture media to form a liquid culture. The resulting culture of eukaryotic cells may be passaged for any number of times. Ultimately, the eukaryotic cells may be processed for another round of cryopreservation.

Novel compositions and methods for the cryopreservation of eukaryotic cells are desirable. It is well documented that the toxicity of the cryoprotectant can be a major limitation to successful cryopreservation of eukaryotic cells. The extreme temperatures that the eukaryotic cells are subjected to during cryopreservation create significant technical hurdles that must be overcome when cryopreserving eukaryotic cells. Major cryoinjuries can occur in relation to the freezing and thawing process, and may include; pH fluctuation, ice crystal formation, osmotic pressure and cryoprotectant toxicity. Accordingly, the development of novel formulations that contain previously uncharacterized cryoprotectant molecules may provide solutions to overcome the technical hurdles associated with the cryopreservation of eukaryotic cells. It should be emphasized that eukaryotic cell types can behave differently when exposed to various cryopreservation compositions and methods. Accordingly, some eukaryotic cell lines are known to be recalcitrant to cryopreservation. So-called recalcitrant lines do not survive drying and freezing during *ex-situ* conservation as these lines cannot resist the effects of drying or temperatures less than 10°C; and cannot be stored for long periods like cell lines of orthodox or non-recalcitrant cells. Where compositions and methods for the cryopreservation of eukaryotic cells may work for one cell line, such methods may not work for other types of eukaryotic cells.

The identification and use of novel compositions and methods for the cryopreservation of eukaryotic cells is essential to improve recovery and storage stability of eukaryotic cells. With the increasing number of gene targeting projects and rapid screening assays utilizing multiple eukaryotic species suspension cultures as an initial starting material, both transgenic and non-transgenic, the ability to reproducibly cryopreserve and store, in addition to, rapidly recovering and re-initiating a wide variety of eukaryotic cells is increasingly the determining factor for the success or failure of such projects. The potential for the use of novel compositions and methods for the cryopreservation of eukaryotic cells is especially important for recalcitrant species. Suspension cultures of cell lines, photo-autotrophic suspension cell lines, plant tissues, and plant structures of such recalcitrant species (especially, those of elite plant cultivars) are currently difficult or incapable of being cryopreserved and stored for extended periods of time.

Therefore, a need exists for development of improved cryopreservation techniques, and to develop compositions of matter that allow for successful cryopreservation and recovery of eukaryotic cells without any alteration (i.e., physiological or genetic modifications) to the eukaryotic cells.

### II. Terms

Unless defined otherwise, all technical and scientific terms used herein have the same meaning as commonly understood by one of ordinary skill in the art to which this disclosure relates. In case of conflict, the present application including the definitions will control. Unless otherwise required by context, singular terms shall include pluralities and plural terms shall include the singular. All publications, patents and other references mentioned herein are incorporated by reference in their entireties for all purposes as if each individual publication or patent application were specifically and individually indicated to be incorporated by reference, unless only specific sections of patents or patent publications are indicated to be incorporated by reference.

In order to further clarify this disclosure, the following terms, abbreviations and definitions are provided.

As used herein, the terms "comprises", "comprising", "includes", "including", "has", "having", "contains", or "containing", or any other variation thereof, are intended to be non-exclusive or open-ended. For example, a composition, a mixture, a process, a method, an article, or an apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive "or" and not to an exclusive "or". For example, a condition A or B is satisfied by any one of the following: A is true (or present) and B is false (or not present), A is false (or not present) and B is true (or present), and both A and B are true (or present).

The term "invention" or "present invention" as used herein is a non-limiting term and is not intended to refer to any single embodiment of the particular invention but encompasses all possible embodiments as disclosed in the application.

As used herein, the term "cryopreservation agent" or "cryoprotectant" is a substance that is used to protect eukaryotic cells (and also tissues, organs) from freezing damage. Further, the cryopreservation agent or cryoprotectant may protect the eukaryotic cells (and also tissues or organs) from cold and heat shock, dehydration, and cryo-toxicity during cryopreservation. The cryopreservation agent or cryoprotectant may be cell penetrating or non-penetrating. Non-limiting examples of cryoprotectants include glycerol, DMSO (dimethyl sulfoxide), propylene glycol, ethylene glycol, acetamide, and methanol. However it should be cautioned that cryoprotectants have some disadvantages in that they can induce protein denaturation at higher temperature and cause cryoprotectant toxicity in cellular systems (like tissues and organs). The toxicity of the cryoprotectants is a major limitation to successful cryopreservation of eukaryotic cells.

It should be emphasized that the cryopreservation agent or cryoprotectant is distinct from an "extender". The extender is typically added to sperm or semen as a liquid diluent to preserve its fertilizing ability. The addition of an extender to semen protects the sperm against possible damage by toxic seminal plasma, as well as providing nutrients and antibiotics to prolong the effectivity of the semen for usage in artificial insemination.

As used herein, the term "cell suspension media" refers to a media or fluid capable of suspending eukaryotic cells. In some embodiments, the cell suspension media provides the nutrients necessary for the growth and maintenance of eukaryotic cells so that the cells can propagate within the cell suspension media. Cell suspension media is a type of "growth media" that is generally used to describe any nutrient media that favors rapid growth of cultured cells.

As used herein, the term "eukaryote" is any organism whose cells contain a nucleus and other organelles enclosed within membranes.

As used herein, the term "plant" refers to any member of the plant kingdom, and refers to plant cells, whole plants, plant organs, plant tissues, seeds and progeny of the same. Plant cells include, without limitation, cells from seeds, suspension cultures, embryos, meristematic regions, callus tissue, leaves, roots, shoots, gametophytes, sporophytes, pollen and microspores. Included within the definition are "monocotyledonous" (any member of the flowering plants, or angiosperms, that has a single leaf, or cotyledon, in the embryo of the seed) and "dicotyledonous" (any member of the flowering plants, or angiosperms, that has a pair of leaves, or cotyledons, in the embryo of the seed) plants. Further, included within the definition of plant are algae, especially the strains commonly used in plant biotechnology research (*Synechocystis, Physcomitrella*, *Chlamydomonas, Synechococcus,* etc.).

As used herein, the term "suspension cell" or "suspension culture" (for example; "plant suspension cell line", "plant suspension culture", "plant suspension cell(s)" or "plant cell suspension culture") are a specialized population of homogenous, undifferentiated plant cells grown in liquid nutrient or culture media. The cells are typically suspended within the media as opposed to adhering to a surface.

As used herein, the term "autotroph", refers to an organism that produces complex organic compounds (carbohydrates, fats, and proteins) from simple inorganic molecules using energy from light (by photosynthesis) or inorganic chemical reactions. They are typically able to make their own food. Some autotrophs can fix carbon dioxide.

As used herein, the term "photo-autotrophic suspension cell line" refers to a plant cell culture that is photosynthetically self-sufficient. Typically, these organisms use photons as an energy source, and are capable of producing complex organic compounds (carbohydrates, fats, and proteins) from simple inorganic molecules, which include carbon dioxide and other non-reduced sources of carbons, such as bicarbonate, using energy from light (by photosynthesis). In principle all phototrophic life forms (especially plant cells) can be used in accordance with the present disclosure. "Phototrophic growth" is growth using light as an energy source, and does not require a reduced carbon source such as a sugar, carbohydrate, organic acid, amino acid, protein, lipid, etc.

As used herein, the term "calli" or "callus" refers to a group of structurally undifferentiated cells derived from any plant parts of plants. Once a plant has been induced to form callus, the callus tissue can be used as an experimental system to investigate and solve a broad range of basic research problems, and to introduce foreign genes into a variety of horticultural and agronomic plants for the purpose of crop improvement.

In monocot tissue culture, embryogenic callus refers to callus that has the potential to regenerate plants. Embryogenic callus is also described as either Type I or Type II. Type I callus is compact, opaque, relatively slow growing, structurally complex, but capable of long-term regeneration either by organogenesis or embryogenesis. Type II callus is friable, rapidly growing, structurally less compact with more clearly formed somatic embryos often consisting of a somatic embryo borne on a suspensor-like structure. Tomes, D. T., p. 175-203. In S. W. J. Bright and M. G. K. (Eds.) Advances in agricultural biotechnology: Cereal tissue and cell culture. Nijhoff/Junk, Boston. Type I callus is the typical callus formed from many maize genotypes, including many of those that are agronomically important. Unfortunately, Type I callus is less amenable to transformation than Type II callus. In plant species other than maize, different terminology, other than Type I and Type II, are used to describe similar callus types.

As used herein, the term "plant tissue" and "plant structure" refer to an organized grouping of differentiated and undifferentiated plant cells. For example, a plant structure would include plant embryos, leaves, pollen, roots, root tips, anther, silks, flowers, kernels, bulbs, tubers, rhizomes and seeds. Further, plant structure includes "plant organs" that constitute plant tissue or a group of tissues that constitute a morphologically and functionally distinct part of a plant. Plant tissues may be located and isolated from a plant or in a plant organ, tissue or cell culture.

As used herein, the term "embryo" or "plant embryo" refers to either a zygotic embryo or a somatic embryo. A zygotic embryo is found inside a botanic seed produced by sexual reproduction. Somatic plant embryos can be produced by culturing embryogenic tissue by standard methods under laboratory conditions in which the cells comprising the tissue are separated from one another and cultured to develop into minute, complete embryos. As used herein, "plant embryo" includes embryos at various stages of development and includes both early-stage and cotyledonary embryos.

As used herein, the term "plasma membrane" or "cell membrane" or "cytoplasmic membrane" is a phospholipid bi-layer membrane that separates the interior of a cell from the outside environment. The plasma membrane may contain embedded proteins. This cellular structure is involved in a variety of cellular processes such as cell adhesion, ion conductivity and cell signaling and serves as the attachment surface for several extracellular structures, including the cell wall, and intracellular cytoskeleton.

As used herein, the term "cell wall" refers to a rigid layer of polysaccharides lying outside the plasma membrane of the cells of plants, fungi, and bacteria. In the algae and higher plants, it consists mainly of cellulose.

As used herein, the terms "polynucleotide", "nucleic acid", and "nucleic acid molecule" are used interchangeably, and may encompass a singular nucleic acid; plural nucleic acids; a nucleic acid fragment, variant, or derivative thereof; and nucleic acid construct (e.g., messenger RNA (mRNA) and plasmid DNA (pDNA)). A polynucleotide or nucleic acid may contain the nucleotide sequence of a full-length cDNA sequence, or a fragment thereof, including untranslated 5' and/or 3' sequences and coding sequence(s). A polynucleotide or nucleic acid may be comprised of any polyribonucleotide or polydeoxyribonucleotide, which may include unmodified ribonucleotides or deoxyribonucleotides or modified ribonucleotides or deoxyribonucleotides. For example, a polynucleotide or nucleic acid may be comprised of single-and double-stranded DNA; DNA that is a mixture of single- and double-stranded regions; single-and double-stranded RNA; and RNA that is mixture of single- and double-stranded regions. Hybrid molecules comprising DNA and RNA may be single-stranded, double-stranded, or a mixture of single- and double-stranded regions. The foregoing terms also include chemically, enzymatically, and metabolically modified forms of a polynucleotide or nucleic acid.

It is understood that a specific DNA refers also to the complement thereof, the sequence of which is determined according to the rules of deoxyribonucleotide base-pairing.

As used herein, the term "gene" refers to a nucleic acid that encodes a functional product (RNA or polypeptide/protein). A gene may include regulatory sequences preceding (5' non-coding sequences) and/or following (3' non-coding sequences) the sequence encoding the functional product.

As used herein, the term "transgene", refers to a chimeric gene comprising a recombinant promoter and an isolated nucleic acid molecule operably linked to the promoter, wherein the chimeric gene is capable of integrating into the germ line of an organism and being expressed.

As used herein, the term "polypeptide" includes a singular polypeptide, plural polypeptides, and fragments thereof. This term refers to a molecule comprised of monomers (amino acids) linearly linked by amide bonds (also known as peptide bonds). The term "polypeptide" refers to any chain or chains of two or more amino acids, and does not refer to a specific length or size of the product. Accordingly, peptides, dipeptides, tripeptides, oligopeptides, protein, amino acid chain, and any other term used to refer to a chain or chains of two or more amino acids, are included within the definition of "polypeptide", and the foregoing terms are used interchangeably with "polypeptide" herein. A polypeptide may be isolated from a natural biological source or produced by recombinant technology, but a specific polypeptide is not necessarily translated from a specific nucleic acid. A polypeptide may be generated in any appropriate manner, including for example and without limitation, by chemical synthesis. Likewise, a polypeptide may be generated by expressing a native coding sequence, or portion thereof, that is introduced into an organism in a form that is different from the corresponding native coding sequence.

As used herein, the term "modification" can refer to a change in a polynucleotide disclosed herein that results in reduced, substantially eliminated or eliminated activity of a polypeptide encoded by the polynucleotide, as well as a change in a polypeptide disclosed herein that results in reduced, substantially eliminated or eliminated activity of the polypeptide. Alternatively, the term "modification" can refer to a change in a polynucleotide disclosed herein that results in increased or enhanced activity of a polypeptide encoded by the polynucleotide, as well as a change in a polypeptide disclosed herein that results in increased or enhanced activity of the polypeptide. Such changes can be made by methods well known in the art, including, but not limited to, deleting, mutating (*e.g.,* spontaneous mutagenesis, random mutagenesis, mutagenesis caused by mutator genes, or transposon mutagenesis), substituting, inserting, down-regulating, altering the cellular location, altering the state of the polynucleotide or polypeptide (*e.g*., methylation, phosphorylation or ubiquitination), removing a cofactor, introduction of an antisense RNA/DNA, introduction of an interfering RNA/DNA, chemical modification, covalent modification, irradiation with UV or X-rays, homologous recombination, mitotic recombination, promoter replacement methods, and/or combinations thereof. Guidance in determining which nucleotides or amino acid residues can be modified, can be found by comparing the sequence of the particular polynucleotide or polypeptide with that of homologous polynucleotides or polypeptides, e.g., yeast or bacterial, and maximizing the number of modifications made in regions of high homology (conserved regions) or consensus sequences.

The term "derivative", as used herein, refers to a modification of a sequence set forth in the present disclosure. Illustrative of such modifications would be the substitution, insertion, and/or deletion of one or more bases relating to a nucleic acid sequence of a coding sequence disclosed herein that preserve, slightly alter, or increase the function of a coding sequence disclosed herein in crop species. Such derivatives can be readily determined by one skilled in the art, for example, using computer modeling techniques for predicting and optimizing sequence structure. The term "derivative" thus also includes nucleic acid sequences having substantial sequence identity with the disclosed coding sequences herein such that they are able to have the disclosed functionalities for use in producing embodiments of the present disclosure.

The term "promoter" refers to a DNA sequence capable of controlling the expression of a nucleic acid coding sequence or functional RNA. In examples, the controlled coding sequence is located 3' to a promoter sequence. A promoter may be derived in its entirety from a native gene, a promoter may be comprised of different elements derived from different promoters found in nature, or a promoter may even comprise rationally designed DNA segments. It is understood by those skilled in the art that different promoters can direct the expression of a gene in different cell types, or at different stages of development, or in response to different environmental or physiological conditions. Examples of all of the foregoing promoters are known and used in the art to control the expression of heterologous nucleic acids. Promoters that direct the expression of a gene in most cell types at most times are commonly referred to as "constitutive promoters". Furthermore, while those in the art have (in many cases unsuccessfully) attempted to delineate the exact boundaries of regulatory sequences, it has come to be understood that DNA fragments of different lengths may have identical promoter activity. The promoter activity of a particular nucleic acid may be assayed using techniques familiar to those in the art.

The term "operably linked" refers to an association of nucleic acid sequences on a single nucleic acid, wherein the function of one of the nucleic acid sequences is affected by another. For example, a promoter is operably linked with a coding sequence when the promoter is capable of effecting the expression of that coding sequence (*e.g.,* the coding sequence is under the transcriptional control of the promoter). A coding sequence may be operably linked to a regulatory sequence in a sense or antisense orientation.

The term "expression", as used herein, may refer to the transcription and stable accumulation of sense (mRNA) or antisense RNA derived from a DNA. Expression may also refer to translation of mRNA into a polypeptide. As used herein, the term "overexpression" refers to expression that is higher than endogenous expression of the same gene or a related gene. Thus, a heterologous gene is "overexpressed" if its expression is higher than that of a comparable endogenous gene.

As used herein, the term "transformation" or "transforming" refers to the transfer and integration of a nucleic acid or fragment thereof into a host organism, resulting in genetically stable inheritance. Host organisms containing a transforming nucleic acid are referred to as "transgenic," "recombinant," or "transformed" organisms.

The terms "plasmid" and "vector", as used herein, refer to an extra chromosomal element that may carry one or more gene(s) that are not part of the central metabolism of the cell. Plasmids and vectors typically are circular double-stranded DNA molecules. However, plasmids and vectors may be linear or circular nucleic acids, of a single- or double-stranded DNA or RNA, and may carry DNA derived from essentially any source, in which a number of nucleotide sequences have been joined or recombined into a unique construction that is capable of introducing a promoter fragment and a coding DNA sequence along with any appropriate 3' untranslated sequence into a cell. In examples, plasmids and vectors may comprise autonomously replicating sequences for propagation in bacterial hosts.

Polypeptide and "protein" are used interchangeably herein and include a molecular chain of two or more amino acids linked through peptide bonds. The terms do not refer to a specific length of the product. Thus, "peptides", and "oligopeptides", are included within the definition of polypeptide. The terms include post-translational modifications of the polypeptide, for example, glycosylations, acetylations, phosphorylations and the like. In addition, protein fragments, analogs, mutated or variant proteins, fusion proteins and the like are included within the meaning of polypeptide. The terms also include molecules in which one or more amino acid analogs or non-canonical or unnatural amino acids are included as can be synthesized, or expressed recombinantly using known protein engineering techniques. In addition, inventive fusion proteins can be derivatized as described herein by well-known organic chemistry techniques.

The term "fusion protein" indicates that the protein includes polypeptide components derived from more than one parental protein or polypeptide. Typically, a fusion protein is expressed from a fusion gene in which a nucleotide sequence encoding a polypeptide sequence from one protein is appended in frame with, and optionally separated by a linker from, a nucleotide sequence encoding a polypeptide sequence from a different protein. The fusion gene can then be expressed by a recombinant host cell as a single protein.

Expression "control sequences" refers collectively to promoter sequences, ribosome binding sites, transcription termination sequences, upstream regulatory domains, enhancers, and the like, which collectively provide for the transcription and translation of a coding sequence in a host cell. Not all of these control sequences need always be present in a recombinant vector so long as the desired gene is capable of being transcribed and translated.

As used herein, the term "cryopreserving" or "cryopreservation" refers to the storage of biological material, e.g. cells, tissues or organs, at temperatures below 4°C. Generally, the intention of the cryopreservation is to maintain the eukaryotic cells in a preserved or dormant state, after which time the eukaryotic cells are returned to a temperature above 4°C for subsequent use. Preferably, the cryopreserving temperature is below 0°C. For example, the cryopreserving temperature may be below 0°C, -5°C, -10°C, -20°C, -60°C, -80°C or greater. Such temperatures can be reached by exposing the eukaryotic cells to liquid nitrogen, liquid helium, carbon dioxide ('dry-ice'), or slurries of carbon dioxide with other solvents. In some embodiments, the cryopreserving temperature is about -20°C, about -80°C or about -180°C.

As used herein, the term "plurality" as used herein refers to two or more than two.

As used herein, the term "suspending" as used herein is intended to include any placement of a solid (e.g., eukaryotic cells) in a liquid whether or not an actual suspension is created. As such, the term "suspending" is intended to include any mixing of a solid in a liquid or any other placement of a solid in a liquid. As a result, the term "suspension" is likewise not intended to be limited to suspensions, but rather is intended to mean any mass having a solid present in a liquid.

As used herein, the term acclimating refers to allowing eukaryotic cells or other biological materials to respond in physiology and temperature to a change in environment. For example the adaptation to a new temperature and media.

As used herein, the term "inoculating" refers to implanting or introducing a eukaryotic cell into a cell suspension media. Inoculate or inoculating a culture of eukaryotic cells in the described culture conditions stated throughout the specification refers to starting a culture of eukaryotic cells in the culture conditions as is commonly used in the art of tissue culture. The eukaryotic cells that are introduced into a cell suspension media may be referred to as "seed", "starter" or "inoculum". "Tissue culture" is a term used to describe the process where plant cells are grown outside of an intact plant in a suitable nutrient media (e.g., cell suspension media). Tissue culture is defined as a method wherein parts of a plant are transferred into an artificial environment in which they can continue to survive. The term tissue culture as understood in the art refers to cultured tissue which may consist of individual or groups of plant cells, protoplasts or whole or parts of a plant organ.

In tissue culture, plant cells can be grown on a solid surface as pale colored lumps known as callus culture or as individual or small clusters of cells known as suspension culture. Cells grown in culture are actively dividing and can be maintained indefinitely in an undifferentiated state by transferring the cells to fresh cell suspension media (subculturing or passaging). Cultured cells may also be induced to re-differentiate into whole plants. Tissue culture is well known in the field of plant biology and has several applications, for example it may be used to produce large quantities of plants or plant material in a short period of time (micropropagation).

Plant tissue cultures can be initiated from almost any part of the source plant (termed explant) although younger parts of the plant are generally more useful as they contain more actively dividing cells. Although tissue culture is well known in the art different plants may vary in the exact conditions required to maintain the cells in culture. Cells in tissue culture are generally different from those in an intact plant. It is also well known in the art that cultured plant cells produce different amounts and altered amounts of metabolites.

The term "passaging" refers to conditions that typically involve harvesting (withdrawing) cells during mid-exponential (mid-log) growth, diluting or splitting the cells at mid-exponential growth with fresh cell suspension media, and cultivating the diluted or split cell culture to mid-exponential growth. The terms "mid-log" and "mid-exponential" as used herein do not necessarily refer to the precise mid-point of exponential growth but rather to a range around the mathematical mid-point. Each round of cultivation to mid-exponential growth is considered one cell passage. Cells to be cryopreserved from suspension can be successfully cryopreserved with only one passage or up to as many as about twenty passages. About two to ten passages are commonly completed. Four to eight passages allows for recovery of cells from a cryopreserved state for re-cultivation.

As used herein, the term "recovering" refers to the isolation of the eukaryotic cells or other biological materials such that it is significantly free of any media component.

As used herein, the term "plant regeneration" refers to the generation of fully differentiated plants from undifferentiated plant tissues, especially callus.

As used herein, the term "freezing" refers to chilling the eukaryotic cells or biological materials, converting the eukaryotic cells or biological materials to a frozen state, and/or maintaining the eukaryotic cells or biological materials in a frozen or chilled state.

As used herein, the term "thawing" refers to the process of incubating the frozen eukaryotic cells or biological materials at an ambient temperature above the freezing temperature until the eukaryotic cells or biological materials have reached said ambient temperature. Generally, as a result of thawing there are no or substantially no ice crystals in all or part of the composition or plurality of eukaryotic cells. Hence the term "thawing" includes complete and partial thawing.

As used herein, the term "diffusion" refers to the intermingling of substances by the natural movement of their particles.

As used herein, the term "flash-freezing" generally refers to a refrigeration process that will quickly freeze any material. It is desirable to be able to quick- freeze many different materials but particularly eukaryotic cells. The application of flash-freezing can be utilized in order to avoid deterioration or to place the material quickly into condition for transport or use. Typically, a plurality of eukaryotic cells can be subjected to flash-freezing by immersing the material in liquid nitrogen (of a temperature of -196°C), carbon dioxide, or helium so that the plurality of eukaryotic cells are immediately frozen.

### III. Embodiments of the Present Disclosure

In an embodiment, the subject disclosure relates to a media composition, comprising a eukaryotic cell suspended within an aqueous solution, the aqueous solution including at least one cryopreservation agent, a cell suspension media, a phosphatidylcholine. In a further embodiment, the aqueous solution includes only a cryopreservation solution. The cryopreservation solution comprising at least one cryopreservation agent and a phosphatidylcholine.

Eukaryotic cells are by definition cells that contain a nucleus and any other cellular organelle enclosed within a membrane. More specifically, the membranes may comprise a plasma membrane. Typical examples of eukaryotic cells include cells originating from the kingdoms of Animalia, Plantae, and Fungi. Any cell type of these kingdoms may be cyropreserved using the compositions and methods of the disclosure.

In an embodiment, the eukaryotic cell is a plant cell. In light of this disclosure, plant cells are subdivided and categorized as monocotyledonous, dicotyledonous, or as algae. The development of novel compositions and methodologies for cryopreservation of plant cells have proven to be difficult. Plant cells and, in particular, plant cells in tissue culture, exhibit an array of heterogeneity with respect to growth rate, doubling time, mitotic index, cell synchrony, nuclear to cytoplasmic ratio and extent of vacuolation. Cells present in any given culture also exhibit a variety of physiological and morphological variations. Plant cell suspensions of different varieties or species require different protocols for cryopreservation. In addition, if performed improperly, cryopreservation can induce the very mutations which the process is attempting to prevent. Surprisingly, the subject disclosure provides novel compositions and methodologies for cryopreservation that can overcome these technical hurdles.

In a further embodiment, the eukaryotic plant cell is a monocotyledonous plant cell. For example, non-limiting monocotyledonous plant cells includes; maize, wheat, rice, black grass, tall fescue, bent grass, and anza wheat. In another embodiment, the eukaryotic plant cell is a dicotyledonous plant cell. A non-limiting listing of dicotyledonous plant cells includes; soybean, tobacco, *Arabidopsis,* canola, cotton, rape, sunflower, and carrot. In a further embodiment, the eukaryotic plant cell is an algae. Exemplary algae plant cells include, but are not limited to, *Synechocystis, Physcomitrella, Chlamydomonas,* and *Synechococcus.*

In other embodiments the subject disclosure relates to a plant cell, plant part, and /or plant structure that may be cryopreserved using the compositions and methods disclosed herein. Any plant species or plant cell may be cryopreserved using the compositions and methods disclosed herein for storage at low-temperature conditions. In some embodiments, plant cells which are cryopreserved using the compositions and methods in accordance with the present disclosure include, but are not limited to cells obtained from, a higher plant, a consumable plant, a crop plant, and a plant utilized for its oils (e.g., an oilseed plant). Such plant cell lines include, for example and without limitation: alfalfa; soybean; cotton; rapeseed (canola); linseed; corn; rice; brachiaria; wheat; safflower; sorghum; sugarbeet; sunflower; tobacco; and grasses (e.g., turf grass). In particular examples, plant cells which are cryopreserved using the compositions and methods in accordance with the present disclosure herein includes, for example and without limitation: *Brassica napus*; Indian mustard (*Brassica juncea*); Ethiopian mustard (*Brassica carinata*); turnip (*Brassica rapa*); cabbage (*Brassica oleracea*); *Glycine max*; *Linum usitatissimum*; *Zea mays*; *Carthamus tinctorius*; *Helianthus annuus*; *Nicotiana tabacum*; *Arabidopsis thaliana*, Brazil nut (*Betholettia excelsa*); castor bean (*Ricinus communis*); coconut (*Cocus nucifera*); coriander (*Coriandrum sativum*); *Gossypium* spp.; groundnut (*Arachis hypogaea*); jojoba (*Simmondsia chinensis*); oil palm (*Elaeis guineeis*); olive (*Olea eurpaea*); *Oryza saliva*; squash (*Cucurbita maxima*); barley (*Hordeum vulgare*); sugarcane (*Saccharum officinarum*); *Triticum* spp. (including *Triticum durum* and *Triticum aestivum*); and duckweed (*Lemnaceae* spp.). In some embodiments, the plant cell may have a particular genetic background, as for elite cultivars, wild-type cultivars, and commercially distinguishable varieties. In other embodiments the plant cell is provided as a plant suspension cell line, or a photo-autotrophic suspension cell line.

In further embodiments, the plant cell is a recalcitrant cell that cannot be preserved via traditional cryopreservation methods that were previously known in the art. It is commonly known that certain plant species and cell lines derived therefrom are recalcitrant to previously established cryopreservation protocols. The recalcitrance is partly due to the sensitivity of the plant cells to the type and concentration of cryoprotectants. There are at least two potential causes of membrane destabilization, chemical toxicity of the cryoprotectants used in the acclimation steps, and extreme dehydration resulting from exposure to the concentrated solutions (e.g., vitrification). Other factors such as ice formation during either cooling or warming, and mechanical disruption of cells due to fracturing of the frozen cryopreservation solution can also contribute to destabilization of membranes.

Furthermore, it is hypothesized that the severe dehydration resulting from the exposure to concentrated solutions of cryoprotectants can cause the structural transitions in a diverse array of biological molecules including lipids, proteins and nucleic acids. Under these conditions, there are several alterations in the ultrastructure of the plasma membrane and other cellular membranes. These changes include the formation of particulate domains in the plasma membrane which result in the removal of water that is associated with the hydrophilic regions of the proteins and lipid components of the membranes. Alternatively, destabilization can also occur by contacting plant cell membranes with a permeablizing cryoprotectant compound (*i.e.,* a compound that fluidizes a channel protein within a plant cell membrane). The development of novel compositions and methods for cryopreservation can minimize membrane destabilization resulting from severe dehydration.

The disclosure is readily applicable for existing and established plant cells that are in use for tissue culture as plant cell lines. In an embodiment, the disclosure relates to tobacco cell lines (e.g., NT-1 and BY-2), rice cell lines (e.g., T309), and soybean cell lines (e.g., Maverick).

In an embodiment, tobacco cell lines such as NT-1 and BY-2 (Kato et al. 1972, Proc. IV IFS: Ferment. Technol. Today 689-695; An, G., 1985 Plant Physiol. 79, 568-570; Nagata et al. 1992, International Review of Cytology 132, 1-30.) are exemplary tobacco plant cells because these lines are particularly susceptible to handling in tissue culture, are readily transformed, and produce stably integrated events; despite being recalcitrant to cryopreservation.

The tobacco cell line, NT-1, was originally developed from *Nicotiana tabacum* L. variety bright yellow 2. The NT-1 cell line is widely used and readily available. Unfortunately, the cell line may be recalcitrant to cryopreservation, and may change or be variable in response to culture conditions. NT-1 cells suitable for use with the composition and methods of the present disclosure are publically available from the American Type Culture Collection under accession number ATCC No. 74840.

The tobacco cell line, BY-2, was originally developed from *Nicotiana tabacum* L. variety bright yellow-2. The BY-2 cell line is used for a broad range of plant studies around the world (Nagata T, Matsuoka K, Inze D (ed) (2006) Biotechnology in agriculture and forestry 58, Tobacco BY-2 cells: from cellular dynamics to omics. Springer-Verlag Berlin Heidelberg). A suspension culture was derived from a callus induced from a stem of *Nicotiana tabacum* L. variety Bright Yellow 2 (Nagata T, Nemoto Y, Hasezawa S (1992) Tobacco BY-2 cell line as the "HeLa" cell in the cell biology of higher plants. International Review of Cytology132: 1-30). The BY-2 cells are fast growing and highly homogeneous. Exemplary BY-2 cells suitable for use in the examples below are available from known public sources.

In an embodiment, soybean cell lines such as *Glycine max (L.) Merr*. variety Maverick (PROLT) are exemplary soybean plant cells because these lines are particularly susceptible to handling in tissue culture, are readily transformed, and produce stably integrated events; despite being recalcitrant to cryopreservation. The first disclosure of the Glycine max (L.) Merr. var. Maverick cell suspension line 'PROLT' was the registration published in Crop Science 38:549 (1998).

In an embodiment, rice cell lines such as variety T309 are exemplary rice plant cells because these lines are particularly susceptible to handling in tissue culture, are readily transformed, and produce stably integrated events; despite being recalcitrant to cryopreservation. The cell suspension lines of this rice variety are known in the art and available from known public sources. For example, the line is described in U.S. Pat. No. 8,012, 752 and Lee. T., et al., Plant Molecular Biology Reporter 22:3; pp. 259-267.

Other plant cell lines are known in the art, and included herein as embodiments of the subject disclosure. For example, the *Zea mays* lines of the Black Mexican Sweet (BMS) variety are maintained as plant cell suspension lines, for which cryopreservation using the methods and composition of matter disclosed herein is desirable (U.S. Pat. No. 8,012,752). In addition, The *Zea mays* variety Hi-II is a plant cell suspension line, for which cryopreservation using the methods and composition of matter disclosed herein is also desirable (U.S. Pat. No. 8,921,112 and International Pat. App. No. WO2005/107437). Further included as working embodiments, are the plant cell suspension lines listed in U.S. Pat. No. 7,785,885, such as rice cell lines, cotton cell lines, *Zea mays* cell lines and rape cell lines. Any of these plant cell suspension lines can be cryopreserved using the methods and composition of matter disclosed herein.

In an embodiment, the eukaryotic cell is a yeast cell. A non-limiting list of yeast cells includes; *Saccharomyces cerevisiae*, *Candida albicans, Debaromyces japonicus, Fellomyces polyborus, Brettanomyces abstinens, Hyphopichia burtonii*, and *Kloeckera brevis.*

In an embodiment, the eukaryotic cell is a fungal cell. A non-limiting list of fungal cells includes; *Dacrymyces deliquescens, Fennellomyces linderi, Cunninghamella blakesleeana, Tolypocladium inflatum, Radiomyces spectabilis*, *Wallemia sebi,* and *Lophodermium seditiosum*.

In an embodiment, the eukaryotic cell is an animal cell. A non-limiting list of animal cells includes: STEM CELLS (i.e., embryonic, fetal, neonatal, adult and their progenitor cells) such as induced pluripotent stem cells, germ stem cells, mesenchymal stem cells, endothelial stem cells, hematopoietic stem cells, neural stem cells, neural crest stem cells, gastrointestinal stem cells; SOMATIC CELLS such as endothelial cells, epithelial cells from stomach and intestine, mammary epithelial cells (i.e., melanocytes), lung epithelial cells, renal epithelial cells, keratinocytes, urothelial cells (i.e., hepatocytes), corneal cells, lens cells, osteoblasts, osteocytes (i.e., odontoblasts), chondrocytes, ligament cells, tendon cells, gland cells (i.e., pituitary gland cells, salivary gland cells, adrenal gland, exocrine and endocrine pancreatic cells, Leydig cells), fat cells, fibroblasts, retinal cells, neurons (i.e., dopaminergic, GABAergic, glutaminergic, cholinergic, adrenergic neurons), glial cells (i.e., astrocytes, oligodendrocytes, Schwann cells), smooth muscle cells, skeletal muscle cells, cardiomyocytes, immune cells (i.e., B-lymphocytes, T- lymphocytes, macrophages, neutrophils, dendritic cells, mast cells, eosinophils, basophils, natural killer cells, M-cells, microglia); CANCER CELL LINES such as HeLa, adenocarcinoma cervix human, CCF-STTG 1, astrocytoma brain human, Hep G2, carcinoma, hepatocellular liver human, SK-MEL-5, melanoma, malignant skin human, Saos-2, osteosarcoma bone human, WERI-Rb-1, retinoblastoma eye, retina human; IMMORTALIZED CELL LINES such as NuLi, human bronchial epithelium, CuFi, human bronchial epithelium, CHON-001, human bone cartilage fibroblast, BJ-5ta, human foreskin fibroblast, hTERT-HME1 (ME16C), human mammary epithelium, hTERT-HPNE, human pancreas duct epithelial-like, hTERT RPE-1, human retinal pigmented epithelium, NeHepLxHT, human liver epithelial-like, THESCs, human endometrium fibroblast-like hybridoma cell lines. Generally, the compositions and methods of the subject disclosure do not relate and are not applicable to spermatazoa or the mature sperm of animal species. In an embodiment, the subject disclosure relates to a eukaryotic cell, wherein the eukaryotic cell is not a sperm cell. Although sperm can be categorized in certain usages as a eukaryotic cell, they are structurally distinct from the above described animal cells (and also distinct from any other eukaryotic cell), as sperm cells possess an acrosome and flagellum. An acrosome is a cellular organelle that develops over the anterior half of the head in the spermatozoa of many animals; it is a cap-like structure derived from the Golgi apparatus. The flagellum of a sperm is composed of microtubules and functions to propel the animal sperm. In an embodiment, the subject disclosure relates to a eukaryotic cell, wherein the eukaryotic cell does not possess an acrosome and flagellum.

In additional embodiments, the disclosure relates to eukaryotic plant cells that are plant suspension cell lines. The suspension cell lines can be derived from a lower plant (e.g., algae), a dicotyledonous plant or a monocotyledonous plant. Furthermore, the suspension cell lines may originate from tissue (e.g., callus) or structures (e.g., embryos) of plant species or from thawed cryopreserved cells of such plant species. As individual suspension cell lines from such differing types of organisms vary in their preferences for growth media constituents, many different cell suspension media (e.g., nutrient media) may be used for induction and proliferation of the cell culture. Methods of sterilization, initiation of callus growth, and suspension growth, as well as suitable nutrient growth media, are well-known to those of ordinary skill in the art, and within the scope of the subject disclosure.

Suspension cell lines are capable of rapid growth rates and varying cell densities (i.e., high cell densities or low cell densities). Initial cultures of various suspension cell lines are sub-cultured by transfer into suitable cell suspension media containing, for example, macro and micro nutrients, organic salts and growth hormones. The suspension cell line cultures are exposed to air and in some instances agitated to aerate the media, or in other instances air may be bubbled in the cell suspension media. The suspension cell line cultures are maintained at a temperature of about 20°C and at a pH that may vary as necessary for the robust growth of the suspension cell line cultures. Such suspension cell line cultures can be harvested by removal of the growth media, for example, by filtration or centrifugation. The cells of the suspension cell line culture with the highest viability are those at the early lag or early cell division growth phases that have recently passed through cell division or mitosis.

Plant cell suspensions as set forth in this disclosure can contain transformed plant cell suspensions derived from a lower plant (e.g., algae), a dicotyledonous plant or a monocotyledonous plant. Non-limiting examples of dicotyledonous plants from which the transformed cell suspensions can be derived are tomato, potato, sweet potato, cassava, legumes, alfalfa, soybean, carrot, strawberry, lettuce, oak, maple, walnut, rose, mint, sunflower, safflower, cotton, tobacco, squash, daisy, canola or cactus. Certain aspects of the disclosure utilize tobacco cells lines such as NT-1 or BY-2, rice cell line such as T309, and soybean cell lines such as Maverick. Where the transformed plant cell suspension is derived from a monocotyledonous plant, plants such as wheat, turf grass, maize or corn, rice, oat, barley, sorghum, orchid, iris, lily, onion, banana, sugarcane, or palm can be used to establish the plant cell suspension. Additionally, cell suspensions can be established from lower plants such as ferns, gymnosperms, conifers, horsetails, club mosses, liver warts, hornworts, mosses, red algaes, brown algaes, gametophytes, sporophytes of pteridophytes, or green algae. An exemplary group of plant cell suspensions useful in the present disclosure are plant cell suspensions derived from soybean, rice, or tobacco plants. The above described plant cell suspension may further be maintained as photo-autotrophic suspension cell lines that do not require additional supplementation of carbon for growth and maintenance. In a further embodiment, the disclosure relates to eukaryotic plant cells that are photo-autotrophic suspension cell lines.

The subject disclosure further relates to plant cells that comprise a plant tissue or a plant structure. Exemplary plant tissues and plant structures can include embryos, meristematic tissues, leaves, pollen, roots, root tips, anther, silks, flowers, kernels, bulbs, tubers, rhizomes, calli, and seeds thereof. The plant cells may be cultivated from existing plant structures such as leaves, roots, bark, stems, rhizomes, callus cells, protoplasts, cell suspensions, organs or organ systems, meristems such as apical meristems, seeds or embryos. These types of plant tissues or plant structures may be obtained from established *in vitro* cultures. Cultures may have been long established or only recently adapted to *in vitro* conditions of, for example, specific temperatures, particular light intensities or special growth or maintenance media. Such plant tissues or plant structures may be maintained as a cell suspension or by growth on semi-solid nutrient media.

The subject disclosure further relates to plant embryos. The preparation of embryogenic tissue for plant propagation, regeneration and transformation is time consuming and labor intensive, especially as it involves manual excision of desired explant tissue. For example, in corn the manual removal of individual immature embryos is a common means for isolating tissue useful for experiments. The manual excision of embryogenic tissues is not only laborious; it is fraught with potentially costly ergonomic issues. The application of novel compositions and methods for the cryopreservation of plant embryos provide a benefit to the art of plant tissue culture, plant propagation, regeneration and transformation.

Initially, the plant embryo is collected as either an immature or mature embryo, typically from a seed or kernel (i.e., zygotic embryo), but in other instances from other tissues sources (i.e., somatic embryos). The tissues are considered embryogenic if the cells of the tissue are capable of cell division such that the amount of tissue increases and/or the tissue that is formed is embryonic tissue. The isolated embryo may be intact or partial and may be accompanied by endosperm material. Each immature embryo, either partial or intact, is useful for the production of callus tissue or cell suspension cultures that can be regenerated into a fertile maize plant.

Somatic embryos are embryos formed in tissue cultures of plants, although there are reports of somatic embryos forming *in vivo*. The structure and morphology of somatic embryos is like that of zygotic embryos from true seed, that is somatic embryos go through the same development stages and may appear to have a globular, heart-shaped or torpedo-shaped morphology. In some instances, somatic embryos lack a seed coat, and also sometimes lack a suspensor. Unless they are specially treated, somatic embryos also lack the dormancy that characterizes true seed. Somatic embryos are clonal in origin and thus multiplication using somatic embryos can have the potential for exceedingly high rates of vegetative increase and is therefore of considerable commercial interest. Somatic embryos also lend themselves to large scale production in bioreactors when grown in liquid culture and this form of propagation is of commercial interest when somatic embryos are transformed to provide novel plant forms.

Somatic embryos have been produced from juvenile somatic tissues, or from cultured stocks of juvenile tissues. For example, the induction of somatic embryos from cultured stem sections is commonly known. Similarly, somatic embryos produced from seedling tissues, such as cotyledons, which are juvenile in a physiological sense, have been prepared. Furthermore, somatic embryos have been prepared using hypocotyl sections. Somatic embryos are suitable for transformation via *Agrobacterium tumefaciens,* microinjection, and particle bombardment techniques.

The development of novel compositions and methods for the cryopreservation of embryonic cells is an attractive option for plant tissue culture. Somatic embryos reportedly provide more stable regenerants than shoots. Another advantage of regeneration systems using somatic embryos is their apparent single cell origin. This means that it is unlikely that regenerants are of chimerical origin. The availability of novel compositions and methods for the cryopreservation of embryogenic plant tissues - especially recalcitrant plant tissues - will permit these plants to be immediately available for usage in laboratory experiments. For example, immature embryonic tissue is an ideal material for transformation protocols. The embryonic tissue is reported to produce more stable regenerants than adventitious shoot tissues. Furthermore, the use of embryonic tissue for transformation may overcome the difficulties with off-types which are inherent with the use of stem internode leaf and microtuber tissue.

Further embodiments of the subject disclosure relate to plant cells that comprise a transgene. Such plant cells may be genetically modified cells, wherein the transgene expresses a recombinant polypeptide of interest. Such a plant cell may be described as a stably transformed plant cell. Transgenes may be stacked with a heterologous nucleic acid encoding a second or third or additional transgene. In an embodiment, a transgenic plant cell contains a recombinant insert (genetic event) that remains unchanged over time and passage number as demonstrated based on genetic analyses such as Southern blots, PCR, or qPCR resulting in no significant changes in phenotype growth rate, or transgene expression levels.

Transgenes are polynucleotides that are introduced into a plant cell, and may be used to confer desired traits on essentially any plant. A wide variety of plants and plant cell systems may be engineered for the desired physiological and agronomic characteristics described herein using a transgene encoding a polynucleotide of interest and various transformation methods. Embodiments herein may use any of the numerous methods for the transformation of plants (and production of genetically modified plants) that are known in the art. Various methods for plant transformation have been developed, including biological and physical transformation protocols for dicotyledenous plants, as well as monocotyledenous plants (See, e.g., Goto-Fumiyuki et al. (1999) Nat. Biotechnol. 17:282-6; Miki et al. (1993) Methods in Plant Molecular Biology and Biotechnology (Glick, B. R. and Thompson, J. E., Eds.), CRC Press, Inc., Boca Raton, FL, pp. 67-88). In addition, vectors and *in vitro* culture methods for plant cell and tissue transformation and regeneration of transgenic plants are described, for example, in Gruber, et al., "Vectors for Plant Transformation" in Method in Plant Molecular Biology and Biotechnology, Glick, et al. , eds; CRC Press pp. 89-119 (1993).

The genetic manipulations of a transgenic plant, herein, may be performed using standard genetic techniques and screening, and may be carried out in any cell that is suitable to genetic manipulation. In some embodiments, a recombinant cell may be any organism or microorganism host suitable for genetic modification and /or recombinant gene expression. In some embodiments, a transgenic cell may be a transgenic plant cell. Standard recombinant DNA and molecular cloning techniques used here are well-known in the art and are described in, for example and without limitation: Sambrook et al. Molecular Cloning: A Laboratory Manual; 2nd edition, 1989; Silhavy et al. (1984) Experiments with Gene Fusions, Cold Spring Harbor Laboratory Press, Cold Spring Harbor, NY; and Ausubel et al. (1987) Current Protocols in Molecular Biology, Greene Publishing Assoc. and Wiley-Interscience, New York, NY.

A transformed plant cell, callus, tissue or plant may be identified and isolated by selecting or screening the engineered plant material for traits encoded by the marker transgenes present on the transforming DNA. For instance, selection can be performed by growing the engineered plant material on semi-solid media containing an inhibitory amount of the antibiotic or herbicide to which the transforming gene construct confers resistance (e.g., the *pat*, *dgt-28, dsm-2, aad-1*, or *aad-12* genes). Further, transformed plants, cells, callus and tissues can also be identified by screening for the activities of any visible marker genes (e.g., the β*-glucuronidase*, *rfp, yfp* or *gfp* genes) that may be present on the recombinant nucleic acid constructs. Such selection and screening methodologies are well known to those skilled in the art.

A transgene may also be introduced into a predetermined area of the plant genome through homologous recombination or via non homologous end joining. Methods to stably integrate a transgene within a specific chromosomal site of a plant cell via homologous recombination or non homologous end joining have been described within the art. For instance, site specific integration as described in US Patent Application Publication No. 2009/0111188 A1 involves the use of recombinases or integrases to mediate the introduction of a donor transgene sequence into a chromosomal target. In addition, International Patent Application No. WO 2008/021207, describes zinc finger mediated-homologous recombination to stably integrate one or more donor transgene sequences within specific locations of the genome. The use of recombinases such as FLP/FRT as described in US Patent No. 6,720,475, or CRE/LOX as described in US Patent No. 5,658,772, can be utilized to stably integrate a transgene sequence into a specific chromosomal site. Finally, the use of meganucleases for targeting a donor transgene into a specific chromosomal location was described in Puchta et al, PNAS USA 93 (1996) pp. 5055-5060). Other various methods for site specific integration within plant cells are generally known and applicable (Kumar et al, Trends in Plant Sci. 6(4) (2001) pp. 155- 159). Furthermore, site-specific recombination systems that have been identified in several prokaryotic and lower eukaryotic organisms may be applied for use in plants. Examples of such systems include, but are not limited too; the R/RS recombinase system from the pSR1 plasmid of the yeast Zygosaccharomyces rouxii (Araki et al. (1985) J. Mol. Biol. 182: 191-203), and the Gin/gix system of phage Mu (Maeser and Kahlmann (1991) Gen. Genet. 230:170-176).

In an embodiment, the subject disclosure relates to a cryopreservation agent mixed with phosphatidylcholine. This chemical molecule, phosphatidylcholine, is a major constituent of cell membranes and pulmonary surfactants. Phosphatidylcholine is more commonly found in the exoplasmic or outer leaflet of cell membranes. It is thought to be transported between membranes within the cell by phosphatidylcholine transfer protein. Phosphatidylcholine also plays a role in membrane-mediated cell signaling and activation of other enzymes.

For the first time, described herein, are compositions and methods that include phosphatidylcholine with a cryopreservation agent for the cryopreservation of eukaryotic cells. The use of phosphatidylcholine with a cryopreservation agent was found to provide surprising benefits for the cryopreservation of eukaryotic cells; especially, recalcitrant cells. The usage of this compound in media solutions as a component of a cryopreservation solution is not taught in the art in sufficient detail. Descriptions for the usage of lecithin (which may include phosphatidylcholine amongst numerous other compounds) as an extender for the freezing of sperm is described (Aires, Viviana A., et al. "In vitro and in vivo comparison of egg yolk-based and soybean lecithin-based extenders for cryopreservation of bovine semen." Theriogenology 60.2 (2003): 269-279; Forouzanfar, M., et al. "In vitro comparison of egg yolk-based and soybean lecithin-based extenders for cryopreservation of ram semen." Theriogenology 73.4 (2010): 480-487; Jeyendran, Rajasingam S., et al. "Cryopreservation of human sperm in a lecithin-supplemented freezing medium." Fertility and sterility 90.4 (2008): 1263-1265; Sudano, Mateus J., et al. "Phosphatidylcholine and sphingomyelin profiles vary in Bos taurus indicus and Bos taurus taurus in vitro-and in vivo-produced blastocysts." Biology of reproduction 87.6 (2012): 130; Hinkovska-Galcheva, V., D. Petkova, and K. Koumanov. "Changes in the phospholipid composition and phospholipid asymmetry of ram sperm plasma membranes after cryopreservation." Cryobiology 26.1 (1989): 70-75; Cabrita, E., et al. "Cryopreservation of fish sperm: applications and perspectives." Journal of Applied Ichthyology 26.5 (2010): 623-635; and, Yildiz, Cengiz, Yusuf Bozkurt, and Ilker Yavas. "An evaluation of soybean lecithin as an alternative to avian egg yolk in the cryopreservation of fish sperm." Cryobiology 67.1 (2013): 91-94.), either as a single ingredient or in combination with other molecules such as lecithin. However, the usage of lecithin in cryopreservation media eukaryotic cells (i.e., bovine embryos) has been reported to significantly reduced survival of the cells after freezing (Pugh, P. A., et al. Theriogenology 50.3 (1998): 495-506).

The compositions of the subject disclosure comprise phosphatidylcholine. The phosphatidylcholine will, in general, be added to the composition (i.e., media composition, cryopreservation solution, etc.) prior to cryopreservation of the eukaryotic cells and/or biological materials. Phosphatidylcholine may be described or referred in the alternative as 1,2-Diacyl-sn-glycero-3-phosphocholine, 3-sn-Phosphatidylcholine, L-α-Lecithin, Azolectin, or PC. The chemical formula of phosphatidylcholine is described as 13% C16:0 (palmitic), 4% C18:0 (stearic), 10% C18:1(oleic), 64% C18:2 (linoleic), and 6% 18:3 (linolenic) with other fatty acids being minor contributors, but the disclosure considers any derivative thereof, or a co-polymer comprising phosphatidylcholine within the scope of the disclosure. In some embodiments, phosphatidylcholine has an average molecular weight in the range of 776 Daltons. Phosphatidylcholine is commercially available (e.g., Sigma-Aldrich) in a variety of different compositions. As used herein, phosphatidylcholine includes any plant derived phosphatidylcholine. Non-limiting examples include soybean L-α-phosphatidylcholine, rape seed L-α-phosphatidylcholine, cotton seed L-α-phosphatidylcholine, or sunflower seed L-α-phosphatidylcholine.

Concentrations of phosphatidylcholine in the composition are an embodiment of the disclosure, and will generally be in the range of about 0.05% to 50% of the total volume of the final solution (i.e., media composition, cryopreservation solution, etc.). Further, exemplary embodiments of phosphatidylcholine for use in the composition will generally be provided at a concentration of 0.03%, 0.04%, 0.05%, 0.06%, 0.07%, 0.08%, 0.09%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.25%, 1.5%, 1.75%, 2%, 2.25%, 2.5%, 2.75%, 3%, 3.25%, 3.5%, 3.75%, 4%, 4.25%, 4.5%, 4.75%, 5%, 5.25%, 5.5%, 5.75%, 6%, 6.25%, 6.5%, 6.75%, 7%, 7.25%, 7.5%, 7.75%, 8%, 8.25%, 8.5%, 8.75%, 9%, 9.25%, 9.5%, 9.75%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or 50%. The above listed concentrations include concentrations which are sufficient for cryopreservation of eukaryotic cells at low temperatures. Further embodiments are desirable for practicing the subject matter of the disclosure. For example, a method for suspending phosphatidylcholine within at least one cryopreservation agent to produce a cryopreservation solution as disclosed herein is an embodiment of the disclosure.

In addition to phosphatidylcholine, the composition (i.e., media composition, cryopreservation solution, etc.) may additionally comprise one or more of the following; a buffer, a cryopreservation agent, an antibiotic, a cell suspension media, a media, an antioxidant, a pH indicator, or any combination thereof. In some embodiments, the phosphatidylcholine is mixed with a cryopreservation agent to produce a cryopreservation solution. In most embodiments, the composition is provided as an aqueous composition, or a substantially aqueous composition.

In regards to the cryopreservation agent, the cryopreservation agent may be either a penetrating cryopreservation agent or a non-penetrating cryopreservation agent, and in some instances can comprise both. Exemplary cryopreservation agents can include; glycerol, DMSO (dimethyl sulfoxide), propylene glycol, ethylene glycol, acetamide, and methanol. In another embodiment, the cryopreservation agent can include fructose, sucrose, glucose, sorbitol, or mannitol. Typically, DMSO and glycerol are the most common cryopreservative agents used for cryopreservation.

Examples of concentrations of cryopreservation agents in the composition will generally be in the range of about 0.5% to 75% of the total volume of the final solution (i.e., media composition). Further, exemplary cryopreservation agents in the composition will generally be provided at a concentration of 0.5%, 0.55%, 0.6%, 0.65%, 0.7%, 0.75%, 0.8%, 0.85%, 0.9%, 0.95%, 1%, 1.25%, 1.5%, 1.75%, 2%, 2.25%, 2.5%, 2.75%, 3%, 3.25%, 3.5%, 3.75%, 4%, 4.25%, 4.5%, 4.75%, 5%, 5.25%, 5.5%, 5.75%, 6%, 6.25%, 6.5%, 6.75%, 7%, 7.25%, 7.5%, 7.75%, 8%, 8.25%, 8.5%, 8.75%, 9%, 9.25%, 9.5%, 9.75%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, or 75%. The above concentrations include concentrations which are sufficient for cryopreservation of eukaryotic cells at low temperatures.

The usage of cryopreservation agents for the cryopreservation of eukaryotic cells can be accomplished with either one of two methods. The first method requires the addition of high concentration of low molecular weight cryopreservation agents that penetrate the cells (i.e., DMSO or glycerol). The second method utilizes high molecular weight non-penetrating cryopreservation agents (i.e., certain polymers like polyvinylpyrrolidone (PVP), dextran, hydroxyethyl starch (HES)) which do not enter the cells, in addition to a lower concentration of penetrating cryopreservation agents. The cryopreservation agent prevents injury to the cell during cryopreservation by preventing the addition of solutes at a multi-molar concentration so that the amount of ice formed within the cell is insufficient to result in damaging cell dehydration. Such a cryopreservation agent, to be useful, must be non-toxic at a high concentration and must freely penetrate the cell. Both glycerol and dimethylsulfoxide (DMSO) have been used for this purpose. Glycerol is remarkably non-toxic at high concentrations, but penetrates cell membranes slowly and is therefore difficult to introduce and remove. Dimethylsulfoxide penetrates rapidly but becomes increasingly toxic as concentrations exceed 1M (about 7%). Accordingly, the identification and discovery of novel compounds and methods that incorporate new compounds to protect eukaryotic cells during cryopreservation are desirable.

The subject disclosure relates to embodiments for a media. In some embodiments the media is a cell suspension media, or an agar media or nutrient growth media. Such media can be used to culture plant cells, animal cells, or yeast cells. Media for agar plates and suspension cultures are based on common plant culture media (Murashige and Skoog; MS) and are described in detail herein (See, **Table** 1). Other tissue culture media are known that, when supplemented appropriately, support plant tissue growth and development, including formation of mature plants from excised meristems or embryos. These tissue culture media can either be purchased as a commercial preparation or custom prepared and modified by those of skill in the art. Examples of such media include, but are not limited to those described by Murashige and Skoog, (1962); Chu et al., (1975); Linsmaier and Skoog, (1965); Uchimiya and Murashige, (1962); Gamborg *et al.,* (1968); Duncan *et al.,* (1985); McCown and Lloyd, (1981); Nitsch and Nitsch, (1969); and Schenk and Hildebrandt, (1972), or derivations of these media supplemented accordingly. Those of skill in the art are aware that cell suspension media and media supplements such as nutrients and growth regulators for use in transformation and regeneration are usually optimized for the particular plant variety of interest. Reagents are commercially available and can be purchased from a number of suppliers (see, for example Sigma Chemical Co., St. Louis, MO and Phytotechnology Laboratories, Shawnee Mission, KS). In an embodiment, the media can be cooled, frozen, or maintained at room temperature. In other embodiments, the media reaches a temperature of about 4°C to about -196°C.

The subject disclosure relates to embodiments of a method for cryopreserving a eukaryotic cell. Generally, the method applies to more than one cell, for example a plurality of eukaryotic cells. Accordingly, the plurality of eukaryotic cells are suspended in a cell suspension media composition. The suspension of the plurality of eukaryotic cells within a media composition (containing a cryopreservative agent and phosphatidylcholine) allows diffusion of media composition molecules into the eukaryotic cell membrane or cell wall. In effect, the eukaryotic cells to be cryopreserved are pretreated with agents that increase cellular viability by removing harmful substances secreted by the cells during growth or cell death from the culture media. Additional agents, for example stabilizers, may be further included within the media compositions. The stabilizers may include substances that may be naturally occurring or artificially produced, and can be introduced directly into the culture media. Stabilizers include anti-oxidants or radical scavenger chemicals that neutralize the very deleterious effects attributable to the presence of active oxygen species and other free radicals. Such substances are capable of damaging cellular membranes, both internal and external membranes, such that cryopreservation and recovery of viable cells are seriously compromised. If these substances are not removed or rendered otherwise ineffective, their effects on viability are cumulative over time and severely limit the practical storage life of cryopreserved cells. Furthermore, as cells die or become distressed, additional harmful substances are released, thereby increasing the damage and death of neighboring cells. Ideally, the eukaryotic cells are grown from late lag to mid log phases (i.e., phases of early cell division) to achieve the greatest survival rates on freezing and thawing. Subjecting the eukaryotic cells to cryopreservation at later times result in cells that exhibit higher degrees of vacuolation and differentiation and are larger in size, thus enhancing the risk of freezing injury and decreasing survival rates on freezing and thawing.

In other embodiments, the suspension of the plurality of eukaryotic cells in a media composition allows for the acclimation of the cells in the media composition containing the cryopreservative agent and phosphatidylcholine. This step increases solute concentration of the eukaryotic cells cytosol by introducing moderate concentrations of cryoprotective agents, generally at between about 0.5 M to about 2 M, or between about 5% to about 20%, by weight, into the cytosol. To minimize the time required for acclimation, the suspension of the plurality of eukaryotic cells in a media composition may be performed at about room temperature, although optimal temperature and other conditions for acclimation will preferably match conditions such as media, light intensities and oxygen levels that maintain a viable cell culture. In the suspension step, single or combinations of cryopreservative agents can be added directly to the incubation media all at once, continuously or in a stepwise fashion. The addition of cryopreservative agents in a stepwise manner is sometimes desired to facilitate delivery of the media composition to eukaryotic cells as it is somewhat more gentle than acclimation of a single cryopreservative agent. Time increments or interval between additions for stepwise cryopreservative agents may range from minutes to hours or more, from about one to about ten minutes, from about one to about five minutes, or from about one or about two minute intervals. The numbers of additions of a cryopreservative agent in a stepwise acclimation procedure is typically whatever is practical and can range from a couple (i.e., 1, 2, or 3) to several hundred. In some embodiments, there are less than about twenty additions, less than about ten additions, and less than five additions. Interval periods and numbers of intervals are easily determined by one of ordinary skill in the art for particular type of cell and cryopreservative agent. The eukaryotic cells are incubated in a solution containing the cryopreservative agent or agents to acclimate intracellular and/or extracellular concentrations of the cryopreservative agent. Incubation times range from minutes to hours as practical. The protective effects of suspending the eukaryotic cells in a cryopreservative agent include, in part, removal of a small, but significant amount of water from the cell. This prepares the cell for subsequent vitrification and/or lyophilization by minimizing the shock of sudden intracellular water loss.

In further embodiments, the plurality of eukaryotic cells are acclimated within the media composition (including a cryopreservative agent and phosphatidylcholine) to a reduced temperature. In an aspect of the embodiment, the plurality of eukaryotic cells and the media composition are acclimated to a reduced temperature. Typically, the plurality of eukaryotic cells to be cryopreserved may be acclimated to a temperature which is reduced from culturing temperatures, but above freezing. This prepares the eukaryotic cells for the cryopreservation process by significantly retarding cellular metabolism and reducing the shock of rapid temperature transitions through some of the more critical temperature changes. Critical temperature ranges are those ranges at which there is the highest risk of cell damage, for example, around the critical temperatures of ice crystal formation. As known to those of ordinary skill in the art, these temperatures vary somewhat depending upon the composition of the solution. For water, the principal component of most cell culture media, ice crystal formation and reformation occurs at about 0°C. This temperature may be significantly less than 0°C depending upon the type and amount of solutes that are included with the water.

Acclimation results in the accumulation of endogenous solutes that decreases the extent of eukaryotic cell dehydration at any given osmotic potential, and contributes to the stabilization of proteins and membranes during extreme dehydration. In addition, cold adaptation interacts synergistically with the vitrifying agents and results in alterations in the liquid conformation of the cellular membranes that increase tolerance to both osmotic exclusions and dehydration, which result where the eukaryotic cells are placed in cryopreservation conditions.

As such, acclimation may be carried out in a stepwise fashion or gradually. Steps may be in decreasing increments of about 0.5°C to about 10°C for a period of time sufficient to allow the eukaryotic cells to acclimate to the lower temperature without causing damage. The temperature gradient, whether gradual or stepwise, is scaled to have cells pass through freezing points as quickly as possible. Eukaryotic cells may be gradually or rapidly acclimated to the reduced temperature. In some instances the acclimation occurs in a step-wise or continuous manner. Techniques for acclimation are well known to those of ordinary skill and include commercially available acclimators. Gradual acclimation comprises reducing incubation temperatures about 1 °C or 0.5°C per hour until the target temperature is achieved. Gradual acclimation is most useful for those cells considered to be most sensitive and difficult to cryopreserve. Stepwise acclimation comprises placing the eukaryotic cells in a reduced temperature for a period of time, and a further reduced temperature for another period of time. These steps are repeated as required. As an embodiment the temperature of the plurality of eukaryotic cells and media composition is lowered for a pre-determined amount of time to a pre-determined temperature to result in a frozen plurality of eukaryotic cells and media composition.

Alternatively, acclimation may be carried out in a rapid or instantaneous fashion. The plurality of eukaryotic cells may be submerged within liquid nitrogen of a temperature of -196°C. Other gases can also be used for, example helium or carbon dioxide. The eukaryotic cells may be submerged by dripping, spraying, injecting or pouring the cells directly into a gas at a cryogenic temperature. Subjecting the cells to such conditions results in an instantaneous reduction of incubation temperatures so that the plurality of eukaryotic cells immediately reaches a lowered temperature. As an embodiment the temperature of the plurality of eukaryotic cells and media composition is lowered by flash-freezing to result in a frozen plurality of eukaryotic cells and media composition.

In other embodiments, the plurality of eukaryotic cells and media composition are frozen to a temperature of less than 4°C. The resulting plurality of eukaryotic cells and media composition that reaches such temperatures can be further cryopreserved. Ideally, the freezing of the eukaryotic cells is sufficiently rapid to prevent the formation of large ice crystals which are detrimental to the cell's survival. The critical freezing time should be measured from the frame of reference of a single cell. The eukaryotic cells may be directly frozen, that is brought directly into contact with an agent already at a cryopreservation temperature. Direct methods include dripping, spraying, injecting or pouring the eukaryotic cells into a cryogenic temperature fluid. The eukaryotic cells may be directly contacted to a chilled solid, such as a frozen steel block. A fluid lowered to a cryopreservation temperature may also be poured directly onto a container of the eukaryotic cells. The direct method also encompasses contacting eukaryotic cells with gases, including air, at a cryogenic temperature. A cryogenic gas, such as a cryogenic stream of nitrogen, carbon dioxide, or helium may be blown directly over or bubbled into a cell suspension. Indirect methods involve placing the eukaryotic cells in a container and contacting the container with a solid, liquid, or gas at cryogenic temperature. Proper containers include, for example, plastic vials, glass vials or ampules which are designed to withstand cryogenic temperatures. Containers for indirect freezing methods do not have to be impermeable to air or liquid. For example, a plastic bag or aluminum foil can be adequate. Furthermore, the container may not necessarily be able to withstand cryogenic temperatures. A plastic vial which cracks, but remain substantially intact under cryogenic temperatures may also be used. The eukaryotic cells may also be frozen by placing a sample of eukaryotic cells on one side of a metal foil while contacting the other side of the foil with a gas, solid, or liquid at cryogenic temperature.

Freezing should be sufficiently rapid to inhibit ice crystal formation. The rate of this freezing may, for example, be slow (e.g. 1-10°C/minute), or fast (above 100°C/min). In some embodiments, the rate of freezing is at least 10°C/minute, at least 20°C/minute, at least 50°C/minute or at least 100°C/minute. In some embodiments, the rate of freezing is between 10°C/minute and 1000°C/minute, between 10°C/minute and 500°C/minute, between 10°C/minute and 100°C/minute, between 10°C/minute and 50°C/minute, or between 10°C/minute and 20°C/minute. In general, the composition comprising the eukaryotic cells will initially be at a temperature about 0°C, e.g. at about 4°C or at ambient temperature. From there, its temperature will be reduced to the cryopreserving temperature, for example in a single, essentially uniform step (i.e. without a significant break).

The cryopreserved eukaryotic cell may be stored at a temperature amenable to cryopreservation for any desired amount of time. In certain embodiments, the cells are stored for at least one day, at least one week, at least one month, or at least one year. In further embodiments, the cells are stored for 1-50 days, 1-12 months or 1-4 years. In some embodiments, the cells are stored for less than 10 years.

The subject disclosure relates to embodiments for methods of recovering the plurality of eukaryotic cells from the media composition. Such embodiments are directed to the thawing of cryopreserved eukaryotic cells. Accordingly, the temperature of the composition (eukaryotic cells suspend in the cryopreservation solution) is raised to a temperature at which there are no ice crystals, or substantially no ice crystals in all or part of the composition or the plurality of eukaryotic cells, including complete and partial thawing.

Proper thawing and recovery is essential to cell survival and to recovery of eukaryotic cells in a condition that is substantially the same as the condition in which they were originally frozen. As the temperature of the cryopreserved eukaryotic cells is increased during thawing, small ice crystals consolidate and increase in size in a process termed devitrification. Large intracellular ice crystals are generally detrimental to cell survival. To prevent devitrification, cryopreserved eukaryotic cells should be thawed as rapidly as possible. The rate of heating may be at least about 5°C per minute to 60°C per minute, at least about 10°C per minute to 60°C per minute, at least about 20°C per minute to 60°C per minute, or at least about 30°C per minute to 60°C per minute. More rapid heating rates of 90°C per minute, 140°C per minute to 200°C or more per minute can also be used. While rapid heating is desired, for example plant cells have reduced ability to survive at incubation temperatures significantly above room temperature. To prevent overheating, the cell temperature should be carefully monitored. Any heating method can be employed including conduction, convection, radiation, electromagnetic radiations or combinations thereof. Conduction methods involve immersion in water baths, placement in heat blocks or direct placement in open flame. Convection methods involve the use of a heat gun or an oven. Radiation methods involve, for example, heat lamps or ovens such as convection or radiation ovens. Electromagnetic radiation involves the use of microwave ovens and similar devices. Some devices may heat by a combination of methods. For example, an oven heats by convection and by radiation. Heating should be terminated as soon as the eukaryotic cells are thawed and the surrounding solutions are in liquid form, which should be above 0°C. Cryopreserved eukaryotic cells are often frozen in the presence of one or more agents that depress the freezing point. When these agents are present, the frozen eukaryotic cells may liquefy at a temperature below 0°C such as at about -10°C, -20°C, -30°C or -40°C. Thawing of the cryopreserved eukaryotic cells may be terminated at any of these temperature or at a temperature above 0°C, and in some examples at a temperature above 4°C. In a further embodiment, the frozen plurality of eukaryotic cells and media composition are thawed. In an aspect of the embodiment, the thawing method comprises the steps of raising the temperature of the frozen plurality of eukaryotic cells and media composition for a pre-determined amount of time to a pre-determined temperature, thereby returning the plurality of eukaryotic cells to the pre-cryopreservation state with minimal damage to the structural integrity of said eukaryotic cell.

The subject disclosure relates to embodiments for methods of culturing the plurality of eukaryotic cells from the media composition. Upon obtaining the thawed eukaryotic cells, the cells can be used as an inoculum for working cells or tissue culture production, and may be maintained by periodic cycling of a callus on an agar plate or via passaging a liquid suspension culture. The cryopreserved eukaryotic cells may be thawed and passed to an agar plate of semi-solid media and cultured one or more times at about 25°C or about room temperature for approximately one to two weeks. The thawed cells can be gradually acclimated to growth conditions to maximize survival. The proliferating cells (e.g., callus) are then teased apart and used to inoculate a flask of liquid suspension media to produce a working cell or production culture. The cryopreserved eukaryotic cells are used as inoculum for production cultures and grown at 25°C or about room temperature with continuous agitation before cultured in liquid suspension media. Cultures are passed approximately every 3-14 days depending on the extent of growth observed and may be split 1:2, 1:3, 1:4, 1:5 or 1:10 at each pass at least 1, 2, 3, 4, 5, 6, 7, 8, 9, or 10 times.

The cultures are grown at ambient temperatures with continuous agitation for a period of 3-14 days for the eukaryotic cell line suspension cultures. In most cases the suspension cultures are passed every 2-7 days with a 1:10 split. During scale-up of the suspension cultures, the cultures are passed at a 1:10 split and are grown for at least seven days. The production cultures are grown for 7-15 days before harvest. Cultures are observed daily for characteristic growth, and samples of production cultures are aseptically removed periodically for microscopic examination and determination of packed cell volume. Cells should increase in density to a packed cell volume (PCV) of approximately 35% and should contain at least 50% healthy viable cells, based on a visual estimate, at the time of harvest. Microscopic examination of the cells should reveal a nucleus visible within the cells.

Aeration may vary depending on the oxygen demand of the culture. Dissolved oxygen should be controlled between approximately 100% to 20%. Agitation speed may vary depending on the oxygen demand but should not to exceed 500 rpm. Controlling pH is typically not necessary. Production cultures may be harvested between 7 and 15 days post inoculation by gravity or vacuum filtration using conventional filtration media. In an embodiment the method comprises: growing the plurality of eukaryotic cells on culture media; inoculating a culture flask containing culture media with the plurality of eukaryotic cells to form a liquid culture and passaging the liquid culture of eukaryotic cells at least 1, 2, 3, 4, 5, 6, 7, 8, 9, or 10 times; recovering said passaged plurality of cells; and adding said recovered plurality of cells to a aqueous solution to form a media composition.

Culturing of the plant cells suspensions may be performed at any scale. In one embodiment, the suspension media may be selected from among rich media, minimal media, or a mineral salts media. The culturing system according to the present disclosure can be cultured in any culture format. For example, batch, fed-batch, semi-continuous, and continuous culture modes may be employed herein.

The culture systems according to the present disclosure are useful at any scale (i.e. volume). Thus, e.g., microliter-scale, centiliter scale, and deciliter scale culture volumes may be used. In addition, larger scale cultures, including cultures greater than .5 Liter or 1 Liter scale can be used. In one embodiment, the fermentation volume will be at or above 1 Liter. In another embodiment, the fermentation volume will be at or above .25 Liters, .5 Liters, .75 Liters, 1 Liters, 2 Liters, 3 Liters, 4 Liters, 5 Liters, 10 Liters, 15 Liters, 20 Liters, 25 Liters, 50 Liters, 75 Liters, 100 Liters, 200 Liters, 50 Liters, 1,000 Liters, 2,000 Liters, 5,000 Liters, 10,000 Liters, 50,000 Liters or 100,000 Liters.

In the present disclosure, growth, fermentation, and/or culturing of the plant cell suspensions is performed within a temperature range permitting survival of the cells, for example a temperature within the range of about 4°C to about 55°C, inclusive.

In additional embodiments, the plant cell may be grown to produce differentiated tissue such as shoots and roots. Ultimately, mature plants can be generated from the cryopreserved cell. In some embodiments, a plurality of plants can be generated. Methodologies for regenerating plants are known to those of ordinary skill in the art and can be found, for example, in: Plant Cell and Tissue Culture, 1994, Vasil and Thorpe Eds. Kluwer Academic Publishers and in: Plant Cell Culture Protocols (Methods in Molecular Biology 111, 1999 Hall Eds Humana Press). Such methodologies (i.e., tissue culture) can include at least one process selected from transformation, callus formation, direct embryogenesis, formation of differentiated plant tissue, formation of at least one mature plant, formation of at least one fertile mature plant, and combinations of these processes.

The cryopreserved plant cells can be regenerated into plants by inoculation in a culture media or grown in any other suitable media, for example soil. In some embodiments, a suitable growth media for higher plants may be any growth media for plants, including, but not limited to, soil, sand, any other particulate media that support root growth (e.g., vermiculite. perlite, etc) or hydroponic culture, as well as suitable light, water and nutritional supplements that facilitate the growth of the higher plant. The plants regenerated therefrom may be further regenerated as a tissue culture, for example, through differentiation of dedifferentiated tissue (calli) or by direct embryogenesis of the extracted immature embryos. Regenerated plants can be grown to maturity to provide mature plants, and in some instances grown to fertile mature plants.

Embodiments of the subject disclosure are further exemplified in the following Examples. It should be understood that these Examples are given by way of illustration only. From the above embodiments and the following Examples, one skilled in the art can ascertain the essential characteristics of this disclosure, and without departing from the spirit and scope thereof, can make various changes and modifications of the embodiments of the disclosure to adapt it to various usages and conditions. Thus, various modifications of the embodiments of the disclosure, in addition to those shown and described herein, will be apparent to those skilled in the art from the foregoing description. Such modifications are also intended to fall within the scope of the appended claims. The following is provided by way of illustration and not intended to limit the scope of the invention.

### EXAMPLES

### Example 1:

A media solution was prepared by initially dissolving one gram of soybean L-α-phosphatidylcholine (Sigma-Aldrich®; St. Louis, MO; catalog no. P7443) in ten milliliters of DMSO. The phosphatidylcholine was only partially dissolved, as visible chunks in the DMSO remained after multiple rounds of vortexing. Next, seven milliliters of glycerol was added to the suspension and vortexed. The addition of the glycerol resulted in the solubilization of the phosphatidylcholine within the aqueous mixture of DSMO and glycerol so that no visible chunks of phosphatidylcholine remained. The resulting media solution was observed to be a homogenous, opaque, milky-white suspension. Next, the media solution was diluted to a final volume of one-hundred milliliters by adding specific plant growth media typically used for plant cell suspensions as provided in Table 1 (for example, rice plant cell growth media for rice suspension cell lines, or tobacco plant cell growth media for tobacco suspension cell lines, or soybean plant cell growth media for soybean suspension cell lines, etc.). An optional step included the application of gentle heat until the media solution came to a boil. The heating of the media solution was performed to solubilize the solution, and, to further, decrease the potential of bacterial and other microbial contaminants. Finally, the media solution was cooled and stored at -20°C until further use.

**Table 1: Specific plant growth media**

| **Species** | **Media ID** | **Name** | **Referenced in Patent:** |
|---|---|---|---|
| Soybean/Wheat/Carrot | OS2352 | NB Dicamba (Liquid) | US8,012,752B2 and US8,206,983B2 |
| Soybean/Wheat/Carrot | OS1442 | NB Dicamba (Solid) | US8,012,752B2 and US8,206,983B2 |
| Tobacco/Soybean/Rice | OS2405 | AA (Liquid) | WO2006/052835 and US7,785,885B2 |
| Tobacco/Soybean/Rice | OS1580 | AA (solid) | WO2006/052835 and US7,785,885B2 |
| Tobacco/Soybean | NT1466 | BY2 VP (Liquid) | WO2006/052835 and US7,785,885B2 |
| Tobacco | NT3671 | BY2 (Liquid) | WO2006/052835 and US7,785,885B2 |
| Tobacco | NT3672 | BY2 (Solid) | WO2006/052835 and US7,785,885B2 |

Additional preparations of the media solutions were prepared to contain 0.5% or 1% soybean L-α-phosphatidylcholine and varying concentrations of DMSO and glycerol (**Table** 2). These novel media solutions were compared to a standard media solution routinely used for plant material preservation that contained; 2 M sucrose, 1 M glycerol, 1 M DMSO, with or without 6 mM L-proline **(Table 3),** and specific plant growth media typically used for maintenance of plant cell suspension lines.

**Table 2: Media solution with 1.0% phosphatidylcholine or 0.5% phosphatidylcholine**

| **Components of 1.0% phosphatidylcholine media solution** | **Amount** |
|---|---|
| 1% soybean L-α-phosphatidylcholine | 1 g |
| DMSO | 10 ml |
| 7% glycerol | 7 ml |
| Cell Suspension Media - plant specific (e.g., OS2352 for soybean) | 83 ml |
| Total Volume | 100 ml |

| **Components of 0.5% phosphatidylcholine media solution** | **Amount** |
|---|---|
| Media solution with 1 % soybean L-α-phosphatidylcholine | 10 ml |
| Cell Suspension Media - plant specific (e.g., OS2352) | 10 ml |
| Total Volume | 20 ml |

**Table 3: Standard media solution**

| **Molar Concentration** | **Component** | **Amount/100 mls** |
|---|---|---|
| **2M** | Sucrose-grade 1 | 68.46 gm |
| **1M** | Glycerol-cell culture tested | 7.37 ml |
| **1M** | DMSO-plant cell culture tested | 7.81 ml |
| **Cell Suspension Media** | plant specific (e.g., OS2352 for soybean) | 45.33 ml |
| **Cell Suspension Media with 6 mM L**-**proline** | plant specific (e.g., NT1466 for tobacco) | 45.33 ml |

### Example 2:

A protocol for a controlled rate of cooling was performed cryopreserve plant material suspended in the media solution. This protocol required mixing an equal volume (i.e., 1:1) of plant suspension cells with each of the media solutions. The homogenous mixture was pretreated for up to one hour at a temperature of approximately 4°C. The homogenous mixture was then dispensed into 4 milliliter cryovials and subjected to a controlled cooling rate of 0.5°C per minute to a final temperature of negative 40°C (i.e., -40°C) in a model 7452 Thermo Forma Cryomed™ (Thermo Fisher Scientific; Waltham, MA).

Next, the frozen samples were thawed to evaluate the plant suspension cells for regrowth. The samples were thawed immediately after freezing in a 45°C water bath. Thawed samples were moved to a laminar flow hood for sterile plating. Each vial was poured onto stacks of sterile #4 Whatman filter paper and placed onto a sterile Petri™ dish. After allowing the cells to drain on the filter papers for approximately two minutes to remove excess cryoprotectant, the top filter paper containing cells was transferred onto solid plates of growth media (specific for the plant species) and incubated in the dark at 28°C. Plates were visually checked daily for regrowth of cells.

### Example 3:

Soybean cell line suspensions of the variety Maverick were cryopreserved in the above described media solutions. There were five cell line suspensions of soybean var. Maverick that were tested. These cell line suspensions were labeled as D547, D548, D549, D550 and D551, and were mixed with the above described media solutions (specific for soybean; **Table 2** and **Table 3)** in equal volumes and advanced for cryopreservation evaluation as described in Example 2. Multiple replications were completed for each line using the standard media solution and the media solution containing 1% soybean L-α-phosphatidylcholine as cryoprotectants. The cell suspension lines were plated onto the solid plates of growth media (specific for soybean; **Table 1)** and incubated in the dark at 28°C for several weeks. The cell suspension lines were periodically inspected for recovery of the soybean cell suspension line as determined by new growth of the cells. The soybean cell suspension lines that were cryopreserved in standard media solution produced little to no regrowth of soybean plant cells **(Table 4).** Only one line that was cryopreserved in the standard media solution showed some spotty growth after 30 days of culturing on the solid plates of growth media. Comparatively, the soybean cell suspension lines that were cryopreserved in media solution containing 1% soybean L-α-phosphatidylcholine resulted in regrowth of all five lines by day twelve (**Table 5).** This observation of robust growth of the soybean cell suspension lines that had been cryopreserved in the media solution containing 1% soybean L-α-phosphatidylcholine was surprising. Especially given that numerous types of media solutions for cryopreservation of the soybean var. Maverick cell suspension lines had been unsuccessfully for regrowth of cell suspension lines.

**Table 4: Evaluation of soybean var. Maverick in standard media solution**

| **Line** | **Observed Recovery of Soybean Cell Line Growth** |
|---|---|
| D547 | No growth observed |
| D548 | No growth observed; Day 30 Spotty growth of only one cell line observed |
| D549 | No growth observed |
| D550 | No growth observed |
| D551 | No growth observed |

**Table 5: Evaluation of soybean var. Maverick in 1 % soybean L-α-phosphatidylcholine media solution**

| **Line** | **Observed Recovery of Soybean Cell Line Growth** |
|---|---|
| D547 | Day 12 Growth of cell line observed |
| D548 | Day 12 Growth of cell line observed |
| D549 | Day 12 Growth of cell line observed |
| D550 | Day 12 Growth of cell line observed |
| D551 | Day 12 Growth of cell line observed |

### Example 4:

Rice cell line suspensions of the variety T309 were cryopreserved in the above described media solutions of Example 1 (specific for rice). There were multiple cell line suspensions of rice var. T309 that were tested (i.e., multiple replicates of line 4221 and line 4086). Initially, these cell line suspensions were mixed with the above described media solutions in equal volumes and advanced for cryopreservation evaluation as described in Example 2. The multiple replications for each line using the standard media solution and the media solution containing 1 % soybean L-α-phosphatidylcholine as cryoprotectants were completed. The cell suspension lines were plated onto the solid plates of growth media (specific for rice; **Table 1)** and incubated in the dark at 28°C for several weeks. The cell suspension lines were periodically inspected for recovery of the rice cell suspension lines as determined by new growth of the cells. The rice cell suspension lines that were cryopreserved in standard media solution produced little to no regrowth of rice plant cells **(Table 6).** Only the 4221 cell line that was cryopreserved in the standard media solution showed regrowth after culturing on the solid plates of growth media. This rice line (line 4221) was bulked for creation of a new working cell line band and assigned a new inventory number of line 4372.

**Table 6: Rice var. T309 recovery in media solutions**

| **Line** | **Recovery** |
|---|---|
| 4086 | No growth observed |
| 4221 | No growth observed for most of the replicates; only one cell line had growth that was observed |

Rice cell suspension var.T309, line 4372 suspensions were cryopreserved in suspension media (specific for rice) containing soybean L-α-phosphatidylcholine. The media suspension was prepared as follows. Five milliliters of glycerol was added to the soybean L-α-phosphatidylcholine powder and vortexed multiple times. The solution resulted in a homogenous, opaque, milky-white suspension. This solution was added to forty-five milliliters of cell suspension growth media (OS2405; **Table 1)** to produce a 1% soybean L-α-phosphatidylcholine solution **(Table 7).** The preparation of media solution did not contain any DMSO added to the stock solution. Rather, the DMSO was incorporated into the 1% soybean L-α-phosphatidylcholine media solution by mixing directly with the standard media solution (as described in Example 1) at equal volumes. This resulted in a lower concentration of the DMSO (3.9%) and a lower concentration of soybean L-α-phosphatidylcholine (.5%). The lower concentration of DMSO and soybean L-α-phosphatidylcholine (.5%) was tested to determine if this concentration would be sufficient for cryoprotection. Further, the media solution was not subjected to heat and boiled as previously described as no bacterial or microbial growth had been observed in the media solutions, thereby indicating that the boiling step was optional and could be eliminated from the procedure. The .5% soybean L-α-phosphatidylcholine media solution was stored at 4°C until further use.

**Table 7: Media solution with 1% phosphatidylcholine**

| **Component** | **ml or mg** | **%** |
|---|---|---|
| Glycerol | 5 ml | 10% |
| 1% soybean L-α-phosphatidylcholine | 500 mg | 1% |
| Plant specific cell suspension media(e.g., OS2405) | 45 ml | BASE |

The 4372 line was frozen using the controlled rate cooling protocol described previously. The media solution was evaluated as a cryopreservative with and without the addition of .5% soybean L-α-phosphatidylcholine. The 4372 line suspensions responded equally to both media solution cryopreservation treatments, and the cell suspension lines showed recovered by day 21 **(Table 8).** Accordingly, the use of soybean L-α-phosphatidylcholine in a media solution resulted in efficient recovery of a rice cell line suspension after cryopreservation, and the lower concentration of DMSO was shown to be effective in the efficient recovery of a rice cell line suspension after cryopreservation.

**Table 8: Evaluation of rice var. T309 line 4372 in 1 % soybean L-α-phosphatidylcholine media solution as compared to standard media solution.**

| **Line** | **Treatment** | **Recovery** |
|---|---|---|
| 4372 | Standard media solution | Day 21 Regrowth |
| 4372 | 1 % soybean L-α-phosphatidylcholine | Day 21 Regrowth |

### Example 5:

Tobacco cell line suspensions of the variety BY2 were cryopreserved in the above described media solutions (specific for tobacco). Furthermore, other media solutions were assessed for use as a cryopreservation media solution. The tobacco cell lines were cryopreserved in BIOXcell™ (IMV Technologies; Maple Grove, MN) and BIOXcell Two Step™ (IMV Technologies). The media solutions of BIOXcell™ and BIOXcell Two Step™ were further modified by the addition of DMSO, DMSO/glycerol, and sucrose **(Table 9).** The tobacco var. BY2 cell line suspensions that were tested with the media solutions included the following lines; 4126, 4148, 4190 and 6155. Each cell line was mixed with the above described media solutions in equal volumes and advanced for cryopreservation evaluation as described in Example 2. Multiple replications were completed for each line.

**Table 9: Media solution for cryopreservation of tobacco var. BY2**

| **Treatments** | **BIOX cell™** | **Cell Line** | **Cell Suspension Media** | **2M Sucrose** | **10% DMSO** | **Total Volume ml** |
|---|---|---|---|---|---|---|
| BIOXcell™ 1:1 | 10 ml | 10 PCV | -- | -- | -- | 20 |
| BIOXcell Two Step™ 1:1 | 10 ml | 10 PCV | -- | -- | -- | 20 |
| BIOXcell™ 1:1 + DMSO | 10 ml | 10 PCV | -- | -- | 2 ml | 22 |
| BIOXcell Two Step™ 1:4 + SUC | 10 ml | 10 PCV | -- | 30 ml | -- | 50 |
| Standard media solution - Control (NT1466) | -- | 10 PCV | 10 ml | 20 ml | -- | 40 |
| 1% soybean L-α-phosphatidylcholine media solution 1:1 | -- | 10 PCV | -- | -- | -- | 20 |

The tobacco cell lines that were cryopreserved in the different media solutions were assayed via 2,3,5-triphenyltetrazolium chloride stain (TTC) that was used to determine the viability of the plant cells. TTC is water-soluble and colorless, and is reduced by living cells to an insoluble red pigment. A working TTC solution was made by dissolving one gram of 2,3,5-triphenyltetrazolium chloride in one-hundred milliliters of de-ionized water, and filter-sterilized using an Steriflip™ (EMD Millipore™; Darmstadt, Germany) disposable vacuum filter unit, and refrigerated at 4°C in a dark bottle. To assay the viability of the tobacco cells, the TTC stain solution was mixed with an equal volume of the sample and allowed to stand overnight at room temperature in the dark. The appearance of red to pink color in the clear TTC stain solution is an indication of cell line viability.

The tobacco var. BY2 cell line suspensions were treated with the TTC stain prior to cryopreservation (pre-freezing of cells) and after being thawed out of cryopreservation (post-freezing of cells). The comparison of TTC stained cell line suspensions pre-freeze to post-freeze showed a loss of cell viability for all four tobacco cell line suspensions that were cryopreserved in only BIOXcell™ and BIOXcell Two Step™ media solutions. The addition of DMSO to the BIOXcell™ media solution improved the viability of cryopreserved tobacco cell line suspensions across all four of the tested lines. Similarly, the addition of sucrose to the BIOXcell Two Step™ media solution improved the viability of cryopreserved tobacco cell line suspensions across three of the four tested lines. The control standard media solution and the 1% soybean L-α-phosphatidylcholine media solution maintained viability of cryopreserved tobacco cell line suspensions in all four cell suspension lines.

Additional assays to test re-initiation and growth of the cryopreserved tobacco cell cultures were performed to validate the cryopreservation protocol. These assays were of interest in assessing recovery from cryopreservation, as cell death within the first 24-72 hours after thawing can occur in significant amounts, thereby decreasing or totally inhibiting recovery and growth. As described in Example 3, the tobacco cell line suspensions were plated onto recovery plates containing tobacco growth media (NT3672; **Table 1**), and were incubated for five days before being evaluated for viability with the TTC staining solution. All four of the tobacco cell line suspensions (i.e., 4126, 4148, 4190, and 6155) cryopreserved in the 1% soybean L-α-phosphatidylcholine media solution were viable after five days of culturing on tobacco growth media. Comparatively, only three of the tobacco cell line suspensions (i.e., 4126, 4148, and 6155) that were cryopreserved in standard media solution were viable after five days of culturing on tobacco growth media. Tobacco cell line suspensions that were cryopreserved in the BIOXcell™ and BIOXcell Two Step™ media solutions did not survive the cryopreservation conditions as compared to the tobacco cell line suspensions that were cryopreserved in 1 % soybean L-α-phosphatidylcholine media solution and the standard media solution. Only tobacco cell line suspension number 4126 that was cryopreserved in BIOXcell Two Step™ containing sucrose (i.e., BIOXcell Two Step™ 1:4 +SUC) was found to be viable as determined by the TTC staining.

The results of the tobacco cell line suspension growth on solid media further supported these results. All four of the tobacco cell line suspensions (i.e., 4126, 4148, 4190, and 6155) cryopreserved in the 1% soybean L-α-phosphatidylcholine media solution were observed to grow robustly on recovery plates containing tobacco growth media (NT3672). The tobacco cell line suspensions for three of the four lines (i.e., 4126, 4148, and 6155) cryopreserved in the standard media solution were observed to grow robustly on recovery plates containing tobacco growth media (NT3672). Little to no growth was observed for the tobacco cell line suspensions that were cryopreserved in the media solutions containing BIOXcell™ and BIOXcell Two Step™ media solutions.

These results demonstrated the successful incorporation of soybean L-α-phosphatidylcholine as a cryoprotectant additive in transgenic tobacco var. BY2 cell line suspensions with no observable deleterious effects on the ability of the cell line suspensions to recover and regrow at a normal rate.

### Example 6:

Additional experiments were completed on tobacco var. BY2 (line NTL4190) cell line suspensions that were cryopreserved in a media solution containing soybean L-α-phosphatidylcholine, tobacco growth media (NT3671; **Table 1**), and 10% glycerol (**Table 10**). The media solutions were mixed as previously described in Example 2 above.

**Table 10: Media solution containing 1% soybean L-α-phosphatidylcholine**

| **Component** | **ml** | **Percentage** |
|---|---|---|
| Glycerol | 5 ml | 10% |
| 1% soybean L-α-phosphatidylcholine | 500 mg | 1% |
| NT3671 | 45 ml | BASE |

The recovery of the tobacco var. BY2 (line NTL4190) cell line suspensions from cryopreservation was evaluated for four different media solution treatments (specific for tobacco). The treatment of tobacco var. BY2 cells using 1% soybean L-α-phosphatidylcholine prepared in 10% glycerol and growth media alone did not result in any regrowth. Interestingly, when 10% DMSO was added to the 1% soybean L-α-phosphatidylcholine prepared in 10% glycerol media solution the tobacco cell suspension lines resulted in robust regrowth in as little as five days after thawing. The addition of the 1% soybean L-α-phosphatidylcholine and 10% glycerol to the standard media solution at a 1:2 ratio also resulted in robust recovery of tobacco cell line suspensions within five days (**Table 11**).

**Table 11: Evaluation of tobacco var. BY2 in 1 % soybean L-α-phosphatidylcholine media solution**

| **Treatment** | **BY2 Tobacco Line** | **Recovery** |
|---|---|---|
| 1% soybean L-α-phosphatidylcholine and 10% glycerol | NTL4190 | 0 |
| 1% soybean L-α-phosphatidylcholine, 10% glycerol, and 10% DMSO | NTL4190 | Day 5 Regrowth |
| 1% soybean L-α-phosphatidylcholine and 10% glycerol mixed with standard media solution at a 1:2 ratio | NTL4190 | Day 5 Regrowth |
| Standard media solution | NTL4190 | Day 5 Spotty Regrowth |

These results demonstrated the successful incorporation of soybean L-α-phosphatidylcholine as a cryoprotectant additive in transgenic tobacco var. BY2 cell line suspensions with no observable deleterious effects on the ability of the cell line suspensions to recover and regrow at a normal rate.

### Example 7:

Soybean cell line suspensions of the variety Maverick were tested for cryopreservation in the BIOXcell™ media solution. Included in the experiment were treatment groups of the following media solutions: BIOXcell™ alone; BIOXcell™ with the addition of 5% DMSO, and 1% soybean L-α-phosphatidylcholine; and a control consisting of the standard media solution cryoprotectant. A single flask of soybean cell suspension was inoculated. On day two of the growth cycle the flask of the soybean cell suspension was split evenly into four sterile 50 ml mini-bioreactor tubes. The soybean cells were allowed to settle at room temperature for five minutes before removing all supernatant. Care was taken not to disturb the cell pellet while removing as much of the media as possible. The packed cell volume (PCV) was measured by using the markings on the tube. An equal volume of 4°C chilled BIOXcell™ and 1% soybean L-α-phosphatidylcholine media solutions were added to each cell pellet. Next, an equal volume of growth media (OS2352; **Table 1**) was added to the control group cell pellet, after the addition of the growth media, the media solution containing the cryoprotectant that was equal to the volume of the cell pellet/growth media mix was added (**Table 12**). The pelleted soybean cell suspensions were re-suspended with gentle shaking by hand. Tubes containing cells and cryoprotectant were placed on ice for one hour. After the one hour pretreatment on ice, samples were evaluated for viability using the TTC stain prior to freezing. Next, a three and a half milliliter aliquot of the cell/media solution mix was placed into sterile four milliliter cryovial using a sterile wide bore disposable pipette.

**Table 12: Treatment groups**

| **Treatment** | **PCV** | **mls Each Treatment** |
|---|---|---|
| BIOXcell™ 1:1 cells | 10 | 10 ml |
| BIOXcell™ + 5% DMSO 1:1 cells | 10 | 10 ml |
| Plant growth media (e.g., OS2352): + equal volume cryoprotectant | 10 | 10 ml (growth media) 20 ml (cryoprotectant) |
| 1% soybean L-α-phosphatidylcholine 1:1 cells | 10 | 10 ml |
| 0.5% soybean L-α-phosphatidylcholine 1:1 cells | 10 | 10 ml |

The cryovials containing the soybean cell suspension and media solution mix were placed in a model 7452 Thermo Forma Cryomed™ for freezing at a controlled rate to 4°C. The vials were maintained at 4°C for fifteen minutes, and then cooled at a rate of 0.5°C per minute to a temperature of negative 40°C (-40°C). The samples were thawed immediately after freezing in a 45°C water bath as described previously. Samples were collected from each vial for TTC viability staining, as previously described above. Next, the samples of cryopreserved soybean cell suspension were poured onto stacks of sterile #4 Whatman filter paper and placed onto sterile Petri™ dishes to remove excess cryoprotectant media solutions. After allowing the cells to drain on the filter papers for approximately two minutes, the top filter paper from each plate, containing the soybean suspension cells was transferred onto solid plates of growth media (OS2352; **Table 1**) and incubated in the dark at 28°C for 24 hours. The viability of the cryopreserved cell suspension lines was observed at 24 hours by removing a small sample of cells with a sterile spatula and placing the cells in TTC stain. After eight days of incubation on solid plates of growth media the viability of the soybean suspension cells was evaluated again and scored for visible growth and compared to the cell lines that were only pretreated on ice for one hour. The visible growth of the soybean suspension cells preserved in 1% and 0.5% soybean L-α-phosphatidylcholine media solutions was comparable to the control samples of soybean suspension cells preserved in the standard media solution. Both groups presented robustly growing soybean suspension cells that were bright yellow in color. Comparatively, the soybean suspension cells preserved in the BIOXcell™ media solutions were pale in color and exhibited little to no visible growth increase.

After an additional twelve days (e.g., twenty total days) of incubation on the filter paper laid atop the plates of solid growth media the soybean suspension cells were scraped off the filter with a sterile spatula onto a fresh plate of solid growth media (OS2352; **Table 1**) and incubated at 28°C for an additional ten days. The growth and viability of the soybean cell suspension lines was visually evaluated. The soybean cell suspension lines that were cryopreserved in the standard media solution (control) and 1% soybean L-α-phosphatidylcholine media solution presented robustly growing suspension cells that were similar in growth rate and color. The soybean cell suspension lines that were cryopreserved in 0.5% soybean L-α-phosphatidylcholine showed significant browning of tissue and less robust growth, but were still viable. The soybean cell suspension lines that were cryopreserved in the BIOXcell™ media solutions groups remained pale in color and showed no visible growth.

### Example 8:

Soybean cell line suspensions of the variety Maverick were tested for cryopreservation in the BIOXcell Two Step™ media solution. Included in the experiment were treatment groups of the following media solutions: BIOXcell™ alone; BIOXcell™ with the addition of DMSO, with the addition of sucrose, with the addition of glycerol, and with the addition of 1% soybean L-α-phosphatidylcholine; BIOXcell Two Step™ alone; BIOXcell Two Step™ with the addition of DMSO, with the addition of sucrose, with the addition of glycerol, and with the addition of 1% soybean L-α-phosphatidylcholine; and a control consisting of the standard media solution cryoprotectant (**Table 13**). The soybean cell suspension were prepared and cryopreserved as described in Example 7. Next, the viability of the soybean cell suspensions was assessed via TTC staining, using the protocol as described above. The soybean cell suspensions were assayed with the TTC stain initially after the cryopreservation treatment, after freezing, and after twenty-four hours of recovery on solid growth media.

**Table 13: Media solution for cryopreservation of soybean var. Maverick**

| **Treatment** | **BIOXcell™ 5X** | **Cell Line** | **Cell Suspension Media** | **2M Sucrose** | **10% DMSO** | **10% Glycerol** | **Total Volume ml** |
|---|---|---|---|---|---|---|---|
| 1:1 | 10ml | 10PCV | -- | -- | -- | -- | 20 |
| 1:4 | 5ml | 7.5PCV | 7.5ml | -- | -- | -- | 20 |
| 1:1 +DMSO | 8ml | 10PCV | -- | -- | 2ml | -- | 20 |
| 1:4 +DMSO/G LYCEROL | 10ml | 12PCV | 13ml | -- | 5ml | 10ml | 50 |
| 1:4 +SUCROS E | 10ml | 10PCV | -- | 30ml | -- | -- | 50 |
| 1:4 +ALL | 10ml | 10PCV | -- | 15ml | 5ml | 10ml | 50 |
| - | 45ml | 60PCV | -- | -- | -- | -- | -- |
| 1:1 | 10ml | 10PCV | -- | -- | -- | -- | 20 |
| 1:4 | 5ml | 7.5PCV | 7.5ml | -- | -- | -- | 20 |
| 1:4 +DMSO | 10ml | 10PCV | 25ml | -- | 5ml | -- | 50 |
| 1:4 + SUCROSE | 10ml | 10PCV | -- | 30ml | -- | -- | 50 |
| 1:4 +ALL | 10ml | 10PCV | -- | 25ml | 5ml | -- | 50 |
| -- | 45ml | -- | -- | -- | -- | -- | -- |
| Standard media solution - Control (e.g.,OS235 2) | -- | 10PCV | 10ml | 20ml | -- | -- | 40 |
| 1:1 1% soybean L-α-phosphatidy lcholine | -- | 10PCV | -- | -- | -- | -- | 20 |
| **TOTAL** | -- | 70PCV | -- | -- | -- | -- | -- |

Viability of the soybean cell suspension lines was assessed, and all of the samples treated in the different cryopreservation media solutions of **Table 13** showed similar results both after treatment and after freezing. However, only five of the soybean cell suspension lines treated in the different cryopreservation media solutions and subjected to twenty hours of culturing on solid growth media showed growth and regeneration. The five cryopreservation treatments that produced robustly growing viable cells were the standard media solution (control), the 1% soybean L-α-phosphatidylcholine media solution, BIOXcell™ with the addition of 5% DMSO media solution, BIOXcell™ with DMSO and 10% glycerol media solution, and BIOXcell Two Step™ with the addition of 2M sucrose media solution (**Table 14**).

**Table 14: Media solution for cryopreservation of soybean var. Maverick and results of TTC staining (i.e., Viable or Non-viable).**

| **Treatments** | **Well** | **Result** |
|---|---|---|
| Post-Treatment -- BIOXcell™ 1:1 | A1 | Viable |
| Post-Treatment -- BIOXcell™ 1:4 | A2 | Viable |
| Post-Treatment -- BIOXcell™ 1:1 +DMSO | A3 | Viable |
| Post-Treatment -- BIOXcell™ 1:4 +DMSO/GLYCEROL | A4 | Viable |
| Post-Treatment -- BIOXcell™ 1:4 +SUCROSE | A5 | Viable |
| Post-Treatment -- BIOXcell™ 1:4 +ALL | A6 | Viable |
| Post-Treatment -- BIOXcell Two Step™ 1:1 | A7 | Viable |
| Post-Treatment -- BIOXcell Two Step™ 1:4 | A8 | Viable |
| Post-Treatment -- BIOXcell Two Step™ 1:4 +DMSO | B1 | Viable |
| Post-Treatment -- BIOXcell Two Step™ 1:4 + SUCROSE | B2 | Viable |
| Post-Treatment -- BIOXcell Two Step™ 1:4 +ALL | B3 | Viable |
| Post-Treatment -- Standard media solution - Control | B4 | Viable |
| Post-Treatment -- 1:1 1% soybean L-α-phosphatidylcholine | B5 | Viable |
| Post-Freezing -- BIOXcell™ 1:1 | C1 | Viable |
| Post-Freezing -- BIOXcell™ 1:4 | C2 | Viable |
| Post-Freezing -- BIOXcell™ 1:1 +DMSO | C3 | Viable |
| Post-Freezing -- BIOXcell™ 1:4 +DMSO/GLYCEROL | C4 | Viable |
| Post-Freezing-BIOXcell™ 1:4 +SUCROSE | C5 | Viable |
| Post-Freezing -- BIOXcell™ 1:4 +ALL | C6 | Viable |
| Post-Freezing -- BIOXcell Two Step™ 1:1 | C7 | Viable |
| Post-Freezing -- BIOXcell Two Step™ 1:4 | C8 | Viable |
| Post-Freezing -- BIOXcell Two Step™ 1:4 +DMSO | D1 | Viable |
| Post-Freezing -- BIOXcell Two Step™ 1:4 + SUCROSE | D2 | Viable |
| Post-Freezing -- BIOXcell Two Step™ 1:4 +ALL | D3 | Viable |
| Post-Freezing -- Standard media solution - Control | D4 | Viable |
| Post-Freezing -- 1:1 1% soybean L-α-phosphatidylcholine | D5 | Viable |
| 24 Hours -- BIOXcell™ 1:1 | E1 | Non-viable |
| 24 Hours -- BIOXcell™ 1:4 | E2 | Non-viable |
| 24 Hours -- BIOXcell™ 1:1 +DMSO | E3 | Non-viable |
| 24 Hours -- BIOXcell™ 1:4 +DMSO/GLYCEROL | E4 | Non-viable |
| 24 Hours -- BIOXcell™ 1:4 +SUCROSE | E5 | Non-viable |
| 24 Hours -- BIOXcell™ 1:4 +ALL | E6 | Non-viable |
| 24 Hours -- BIOXcell Two Step™ 1:1 | E7 | Non-viable |
| 24 Hours -- BIOXcell Two Step™ 1:4 | E8 | Non-viable |
| 24 Hours -- BIOXcell Two Step™ 1:4 +DMSO | F1 | Non-viable |
| 24 Hours -- BIOXcell Two Step™ 1:4 + SUCROSE | F2 | Viable |
| 24 Hours -- BIOXcell Two Step™ 1:4 +ALL | F3 | Non-viable |
| 24 Hours -- Standard media solution - Control | F4 | Viable |
| 24 Hours -- 1:1 1% soybean L-α-phosphatidylcholine | F5 | Viable |

The results described in this Example demonstrate the successful use of soybean L-α-phosphatidylcholine as a media solution for cryoprotection of soybean var. Maverick suspension cell lines. The soybean suspension cell lines that were treated with the cryopreservation solution showed no negative effects in ability of the suspensions to recover and regrow at a normal rate after freezing. The addition of 1% soybean L-α-phosphatidylcholine to the media solution as a cryoprotectant treatment resulted in robust growth of the soybean var. Maverick suspension line at levels equal or better than growth rates demonstrated using the standard media solution cryoprotectant. The implementation of this effective cryopreservative additive - phosphatidylcholine - is a novel and effective plant material cryogenic preservative.

While aspects of this invention have been described in certain embodiments, they can be further modified within the spirit and scope of this disclosure. This application is therefore intended to cover any variations, uses, or adaptations of embodiments of the invention using its general principles. Further, this application is intended to cover such departures from the present disclosure as come within known or customary practice in the art to which these embodiments pertain and which fall within the limits of the appended claims.

## Claims

1. A media composition, comprising:
(i) an aqueous solution including at least one cryopreservation agent, a cell suspension media, and a phosphatidylcholine; and,
(ii) a eukaryotic cell.

2. The media composition of claim 1, wherein the cryopreservation agent is selected from the group consisting of glycerol, DMSO (dimethyl sulfoxide), propylene glycol, ethylene glycol, acetamide, and methanol.

3. The media composition of claim 1, wherein the cryopreservation agent is provided at concentrations from 0.5% to 75% of the total volume of the media composition.

4. The media composition of claim 1, wherein the cell suspension media comprises a monocotyledonous plant cell suspension media or a dicotyledonous plant cell suspension media.

5. The media composition of claim 1, wherein the eukaryotic cell comprises a plant cell.

6. The media composition of claim 5, wherein the plant cell is selected from the group consisting of a monocotyledonous plant cell and a dicotyledonous plant cell.

7. The media composition of claim 6, wherein the monocotyledonous plant cell is selected from the group consisting of maize, wheat, rice, black grass, tall fescue, bent grass, and anza wheat and/or the dicotyledonous plant cell is selected from the group consisting of soybean, tobacco, Arabidopsis, canola, cotton, rape, sunflower, and carrot.

8. The media composition of claim 5, wherein the plant cell comprises a plant suspension cell line, or a photo-autotrophic suspension cell line.

9. The media composition of claim 6, wherein the plant cell comprises a plant tissue or a plant structure.

10. The media composition of claim 9, wherein the plant tissue or the plant structure is selected from the group consisting of embryos, meristematic tissues, leaves, pollen, roots, root tips, anther, silks, flowers, kernels, bulbs, tubers, rhizomes, calli, and seeds thereof.

11. The media composition of claim 5, wherein the plant cell comprises a trans gene.

12. The media composition of claim 1, wherein the phosphatidylcholine is selected from the group consisting of soybean L-α-phosphatidylcholine, rape seed L-α-phosphatidylcholine, cotton seed L-α-phosphatidylcholine, and sunflower seed L-α-phosphatidylcholine.

13. The media composition of claim 1, wherein the phosphatidylcholine is provided at concentrations from 0.05% to 50% of the total volume of the media composition.

14. The media composition of claim 1, wherein the media composition is cooled or frozen.

15. The media composition according to claim 14, wherein the media composition comprises a temperature of about 4°C to about -196°C.
